# EUROPEAN PATENT APPLICATION

(11) **EP 4 342 901 A1**
(43) Date of publication of application: **27.03.2024**
(21) Application number: 22803949.1
(22) Date of filing: 17.05.2022
(51) Int. Cl.: C07F 9/6512, A61K 31/675, A61P 25/28, A61P 39/06

(54) **COMPOUND CONTAINING PHOSPHATE GROUP, PHARMACEUTICAL COMPOSITION CONTAINING SAME, PREPARATION METHOD THEREFOR AND USE THEREOF**

(30) Priority: 20.05.2021 CN 202110555984
(71) Applicant: Shanghai Raising Pharmaceutical Co., Ltd., Pudong New District Shanghai 201321 (CN)
(72) Inventor: ZHANG, Huan, Shanghai 201321 (CN); JING, Hefeng, Shanghai 201321 (CN); MENG, Lijuan, Shanghai 201321 (CN)
(74) Representative: Nederlandsch Octrooibureau
(86) International application number: PCT/CN2022/093265
(87) International publication number: WO 2022/242629

(57) **Abstract**

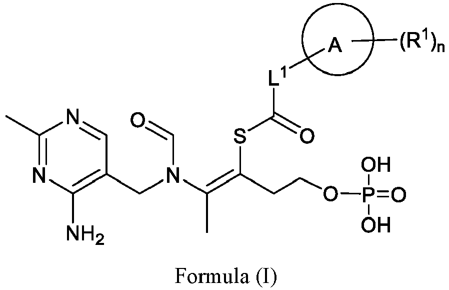

The present invention relates to a compound represent by formula (I), a pharmaceutical composition containing same, a preparation method therefor and a use thereof for the treatment of neurodegenerative diseases.

## Description

### FIELD OF THE INVENTION

The present invention relates to a compound containing a phosphate ester group, a pharmaceutical composition comprising the same, a preparation method therefor and a use thereof for the treatment of neurodegenerative diseases.

### BACKGROUND OF THE INVENTION

Alzheimer's disease (commonly known as senile dementia, AD) is a progressive neurodegenerative disease with cognitive and behavioral abnormalities as the main clinical manifestations, and is the most common form of dementia in the elderly. It is mainly characterized by impairments in recognition abilities and a rapid decline in memory function. The principal pathological physiologic features are the deposition of β-amyloid (Aβ) in the brain forming senile plaques, hyperphosphorylation of tau protein leading to neurofibrillary tangles, disturbances in cerebral glucose metabolism, and loss of neurons/synapses. Due to the disease's long duration and the patient's poor self-care ability, it brings serious mental and economic burdens to families and society. However, there is currently no medication available worldwide that can prevent or delay the progression of the disease. The drugs for the treatment of AD currently on the market are only symptomatic treatment drugs, which can control or improve cognitive and functional symptoms for a period of time but cannot stop or prevent the deterioration of the condition.

### SUMMARY OF THE INVENTION

The present invention provides compounds containing a phosphate ester group which can be used for the prevention or treatment of neurodegenerative diseases. Furthermore, the compounds of the present invention also possess superior properties, including better physicochemical properties (e.g., solubility, physical and/or chemical stability), improved pharmacokinetic properties (e.g., improved bioavailability, suitable half-life and duration of action), improved safety (lower toxicity (e.g., reduced cardiotoxicity) and/or fewer side effects), less resistance, etc.

One aspect of the present invention provides a compound or a pharmaceutically acceptable salt, ester, stereoisomer, tautomer, polymorph, solvate, metabolite, isotopically labeled compound or prodrug thereof, wherein the compound has the structure of Formula (I): 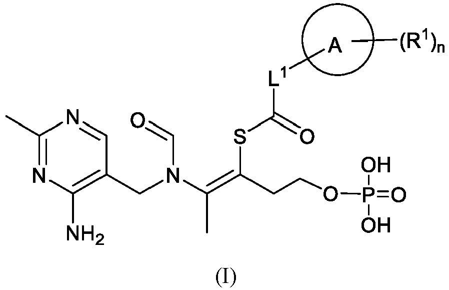 wherein:
ring A is a C₆₋₁₀ aromatic ring or 5- to 14-membered heteroaromatic ring;
L¹ is a direct bond or -R²-C₁₋₆ alkylene-;
R² is -O-, -NH-, -S-, -S(=O)- or -S(=O)₂-;
R¹, at each occurrence, is each independently selected from the group consisting of halogen, hydroxyl, oxo, amino, cyano, nitro, C₁₋₆ alkyl, C₂₋₆ alkenyl, C₂₋₆ alkynyl, C₃₋₆ cyclic hydrocarbyl, 3- to 10-membered heterocyclyl, C₆₋₁₀ aryl, 5- to 14-membered heteroaryl, C₆₋₁₂ aralkyl, =N-OR³, -C(=NH)NH₂, -C(=O)R³, -OC(=O)R³, -C(=O)OR³, -OR³, -SR³, -S(=O)R³, -S(=O)₂R³, -S(=O)₂NR³R⁴, -NR³R⁴, -C(=O)NR³R⁴, -NR³-C(=O)R⁴, -NR³-C(=O)OR⁴, -NR³-S(=O)₂-R⁴, -NR³-C(=O)-NR³R⁴, -C₁₋₆ alkylene-NR³R⁴, -O-C₁₋₆ alkylene-NR³R⁴ and -C₁₋₆ alkylene-O-C₁₋₆ alkyl; or when n is greater than 1, two R¹ together with the group to which they are attached form a C₃₋₆ hydrocarbon ring, 3- to 10-membered heterocycle, C₆₋₁₀ aromatic ring or 5- to 14-membered heteroaromatic ring;
R³ and R⁴, at each occurrence, are each independently selected from the group consisting of H, C₁₋₆ alkyl, C₃₋₁₀ cyclic hydrocarbyl, 3- to 10-membered heterocyclyl, C₆₋₁₀ aryl, 5- to 14-membered heteroaryl and C₆₋₁₂ aralkyl;
the above alkyl, alkylene, alkenyl, alkynyl, cyclic hydrocarbyl, hydrocarbon ring, heterocyclyl, heterocycle, aryl, aromatic ring, heteroaryl, heteroaromatic ring and aralkyl, at each occurrence, are each optionally substituted with one or more substituents independently selected from the group consisting of: halogen, hydroxyl, oxo, amino, cyano, nitro, C₁₋₆ alkyl, C₂₋₆ alkenyl, C₂₋₆ alkynyl, C₃₋₆ cyclic hydrocarbyl, 3- to 10-membered heterocyclyl, C₆₋₁₀ aryl, 5- to 14-membered heteroaryl, C₆₋₁₂ aralkyl, =N-OR⁵, -C(=NH)NH₂, -C(=O)R⁵, -OC(=O)R⁵, -C(=O)OR⁵, -OR⁵, -SR⁵, -S(=O)R⁵, -S(=O)₂R⁵, -S(=O)₂NR⁵R⁶, -NR⁵R⁶, -C(=O)NR⁵R⁶, -NR⁵-C(=O)R⁶, -NR⁵-C(=O)OR⁶, -NR⁵-S(=O)₂-R⁶, -NR⁵-C(=O)-NR⁵R⁶, -C₁₋₆ alkylene-NR⁵R⁶, -O-C₁₋₆ alkylene-NR⁵R⁶ and -C₁₋₆ alkylene-O-C₁₋₆ alkyl, the alkyl, alkenyl, alkynyl, cyclic hydrocarbyl, heterocyclyl, aryl, heteroaryl and aralkyl are further optionally substituted with one or more substituents independently selected from the group consisting of: halogen, hydroxyl, oxo, amino, cyano, nitro, C₁₋₆ alkyl, C₃₋₆ cyclic hydrocarbyl, 3- to 10-membered heterocyclyl, C₆₋₁₀ aryl, 5- to 14-membered heteroaryl and C₆₋₁₂ aralkyl;
R⁵ and R⁶, at each occurrence, are each independently selected from the group consisting of H, C₁₋₆ alkyl, C₃₋₁₀ cyclic hydrocarbyl, 3- to 10-membered heterocyclyl, C₆₋₁₀ aryl, 5- to 14-membered heteroaryl and C₆₋₁₂ aralkyl; and
n is an integer of 0, 1, 2, 3 or 4;
provided that, when L' is a direct bond, ring A is not a benzene ring.

Another aspect of the present invention provides a pharmaceutical composition comprising a prophylactically or therapeutically effective amount of the compound of the present invention or a pharmaceutically acceptable salt, ester, stereoisomer, tautomer, polymorph, solvate, metabolite, isotopically labeled compound or prodrug thereof and one or more pharmaceutically acceptable carriers.

Another aspect of the present invention provides use of the compound of the present invention or a pharmaceutically acceptable salt, ester, stereoisomer, tautomer, polymorph, solvate, metabolite, isotopically labeled compound or prodrug thereof or the pharmaceutical composition of the present invention in the manufacture of a medicament for the prophylaxis or treatment of a neurodegenerative disease or the alleviation of a symptom of the neurodegenerative disease.

Another aspect of the present invention provides the compound of the present invention or a pharmaceutically acceptable salt, ester, stereoisomer, tautomer, polymorph, solvate, metabolite, isotopically labeled compound or prodrug thereof or the pharmaceutical composition of the present invention for use in the prophylaxis or treatment of a neurodegenerative disease or the alleviation of a symptom of the neurodegenerative disease.

Another aspect of the present invention provides a method for the prophylaxis or treatment of a neurodegenerative disease or the alleviation of a symptom of the neurodegenerative disease, wherein the method comprises administering to a subject in need thereof an effective amount of the compound of the present invention or a pharmaceutically acceptable salt, ester, stereoisomer, tautomer, polymorph, solvate, metabolite, isotopically labeled compound or prodrug thereof or the pharmaceutical composition of the present invention.

Another aspect of the present invention provides a method for preparing the compound of the present invention.

### DETAILED DESCRIPTION OF THE INVENTION

### Definition

Unless otherwise defined in the context, all technical and scientific terms used herein are intended to have the same meaning as commonly understood by a person skilled in the art. References to techniques employed herein are intended to refer to the techniques as commonly understood in the art, including variations on those techniques or substitutions of equivalent techniques which would be apparent to a person skilled in the art. While it is believed that the following terms will be readily understood by a person skilled in the art, the following definitions are nevertheless put forth to better illustrate the present invention.

The terms "contain", "include", "comprise", "have", or "relate to", as well as other variations used herein are inclusive or open-ended, and do not exclude additional, unrecited elements or method steps.

As used herein, the term "alkylene" refers to a saturated divalent hydrocarbyl, preferably refers to a saturated divalent hydrocarbyl having 1, 2, 3, 4, 5 or 6 carbon atoms, e.g., methylene, ethylene, propylene or butylene.

As used herein, the term "alkyl" is defined as a straight or branched saturated aliphatic hydrocarbon. In some embodiments, alkyl has 1-12, e.g., 1-6, carbon atoms. For example, as used herein, the term "C₁₋₆ alkyl" refers to a linear or branched group having 1-6 carbon atoms (such as methyl, ethyl, n-propyl, isopropyl, n-butyl, isobutyl, sec-butyl, *tert*-butyl, n-pentyl, or n-hexyl), which is optionally substituted with one or more (e.g., 1 to 3) suitable substituents such as halogen (in which case the group may be referred to as "haloalkyl") (e.g., CF₃, C₂F₅, CHF₂, CH₂F, CH₂CF₃, CH₂Cl or -CH₂CH₂CF₃ *etc*.). The term "C₁₋₄ alkyl" refers to a linear or branched aliphatic hydrocarbon chain having 1-4 carbon atoms (i.e., methyl, ethyl, n-propyl, isopropyl, n-butyl, isobutyl, sec-butyl or *tert*-butyl).

As used herein, the term "alkenylene" refers to a divalent hydrocarbyl containing one or more double bonds, which preferably has 2, 3, 4, 5 or 6 carbon atoms, such as ethenylene, propylene or allylene.

As used herein, the term "alkenyl" refers to a linear or branched monovalent hydrocarbyl having a double bond and 2-6 carbon atoms ("C₂₋₆ alkenyl"). The alkenyl is e.g., vinyl, 1-propenyl, 2-propenyl, 2-butenyl, 3-butenyl, 2-pentenyl, 3-pentenyl, 4-pentenyl, 2-hexenyl, 3-hexenyl, 4-hexenyl, 5-hexenyl, 2-methyl-2-propenyl and 4-methyl-3-pentenyl. When the compound of the present invention contains an alkenylene or an alkenyl group, the compound may exist as the pure E (entgegen) form, the pure Z (zusammen) form, or any mixture thereof.

As used herein, the term "alkynyl" refers to a monovalent hydrocarbyl containing one or more triple bond, and preferably having 2, 3, 4, 5 or 6 carbon atoms, e.g., ethynyl or propynyl.

As used herein, the terms "cyclic hydrocarbylene", "cyclic hydrocarbyl" and "hydrocarbon ring" refer to a saturated (i.e., "cycloalkylene" and "cycloalkyl") or unsaturated (i.e., having one or more double and/or triple bonds in the ring) monocyclic or polycyclic hydrocarbon ring having e.g., 3-10 (suitably having 3-8, and more suitably having 3-6) ring carbon atoms, including but not limited to cyclopropyl(ene) (ring), cyclobutyl(ene) (ring), cyclopentyl(ene) (ring), cyclohexyl(ene) (ring), cycloheptyl(ene) (ring), cyclooctyl(ene) (ring), cyclononyl(ene) (ring), cyclohexenyl(ene) (ring), and the like.

As used herein, the term "cycloalkyl" refers to a saturated or unsaturated, non-aromatic, monocyclic or polycyclic (e.g., bicyclic) hydrocarbon ring (e.g., monocyclic, such as cyclopropyl, cyclobutyl, cyclopentyl, cyclohexyl, cycloheptyl, cyclooctyl, or cyclononyl, or bicyclic, including spiro, fused or bridged cyclic system (such as bicyclo[1.1.1]pentyl, bicyclo[2.2.1]heptyl, bicyclo[3.2.1]octyl or bicyclo[5.2.0]nonyl, or decahydronaphthalene *etc*.))*,* which is optionally substituted with one or more (e.g., 1 to 3) suitable substituents. The cycloalkyl has 3 to 15 carbon atoms. For example, the term "C₃₋₆ cycloalkyl" refers to a saturated or unsaturated, non-aromatic, monocyclic or polycyclic (e.g., bicyclic) hydrocarbon ring having 3 to 6 ring forming carbon atoms (e.g., cyclopropyl, cyclobutyl, cyclopentyl, or cyclohexyl), which is optionally substituted with one or more (e.g., 1 to 3) suitable substituents, e.g., methyl substituted cyclopropyl.

As used herein, the terms "heterocyclyl", "heterocyclylene" and "heterocycle" refer to a saturated (i.e., heterocycloalkyl) or partially unsaturated (i.e., having one or more double and/or triple bonds in the ring) cyclic group having e.g. 3-10 (suitably having 3-8, and more suitably having 3-6) ring atoms, wherein at least one ring atom is a heteroatom selected from the group consisting of N, O and S, and the remaining ring atoms are C. For example, "3- to 10-membered heterocyclyl(ene)" of "3- to 10-membered heterocycle" refers to saturated or partially unsaturated heterocyclyl(ene) or heterocycle having 2-9 (e.g., 2, 3, 4, 5, 6, 7, 8 or 9) ring carbon atoms and one or more (e.g., 1, 2, 3, or 4) heteroatoms independently selected from the group consisting of N, O and S. Examples of heterocyclylene, heterocyclyl and heterocycle include, but are not limited to oxiranyl(ene), aziridinyl(ene), azetidinyl(ene), oxetanyl(ene), tetrahydrofuranyl(ene), dioxolinyl(ene), pyrrolidinyl(ene), pyrrolidonyl(ene), imidazolidinyl(ene), pyrazolidinyl(ene), pyrrolinyl(ene), tetrahydropyranyl(ene), piperidinyl(ene), morpholinyl(ene), dithianyl(ene), thiomorpholinyl(ene), piperazinyl(ene) or trithianyl(ene). Said group also encompasses a bicyclic system, including a spiro, fused, or bridged system (e.g., 8-azaspiro[4.5]decane, 3,9-diazaspiro[5.5]undecane, 2-azabicyclo[2.2.2]octane, *etc*.). Heterocyclylene, heterocyclyl and heterocycle may optionally be substituted with one or more (e.g. 1, 2, 3 or 4) suitable substituents.

As used herein, the terms "aryl(ene)" and "aromatic ring" refer to an all-carbon monocyclic or fused-ring polycyclic aromatic group having a conjugated π electron system. For example, as used herein, the terms "C₆₋₁₀ aryl(ene)" and "C₆₋₁₀ aromatic ring" refer to an aromatic group containing 6 to 10 carbon atoms, such as phenyl(ene) (benzene ring) or naphthyl(ene) (naphthalene ring). Aryl(ene) or aromatic ring is optionally substituted with one or more (such as 1 to 3) suitable substituents (e.g., halogen, -OH, -CN, -NO₂, and C₁₋₆ alkyl, *etc*.).

As used herein, the terms "heteroaryl(ene)" and "heteroaromatic ring" refer to a monocyclic, bicyclic or tricyclic aromatic ring system having 5, 6, 8, 9, 10, 11, 12, 13 or 14 ring atoms, particularly 1 or 2 or 3 or 4 or 5 or 6 or 9 or 10 carbon atoms, and containing at least one heteroatom (such as O, N, or S), which can be same to different. Moreover, in each case, it can be benzo-fused. In particular, "heteroaryl(ene)" or "heteroaromatic ring" is selected from the group consisting of thienyl(ene), furyl(ene), pyrrolyl(ene), oxazolyl(ene), thiazolyl(ene), imidazolyl(ene), pyrazolyl(ene), isoxazolyl(ene), isothiazolyl(ene), oxadiazolyl(ene), triazolyl(ene), thiadiazolyl(ene) *etc.,* and benzo derivatives thereof; or pyridinyl(ene), pyridazinyl(ene), pyrimidinyl(ene), pyrazinyl(ene), triazinyl(ene), etc., and benzo derivatives thereof.

The term "aralkyl" preferably means aryl substituted alkyl, wherein aryl, and alkyl are as defined herein. Normally, the aryl group may have 6-14 carbon atoms, and the alkyl group may have 1-6 carbon atoms. Exemplary aralkyl group includes, but is not limited to, benzyl, phenylethyl, phenylpropyl, phenylbutyl.

As used herein, the term "halo" or "halogen" are defined to include F, Cl, Br, or I.

As used herein, the term "alkylthio" means an alkyl group as defined above attached to a parent molecular moiety through a sulfur atom. Representative examples of C₁₋₆ alkylthio include, but are not limited to, methylthio, ethylthio, tert-butylthio and hexylthio.

As used herein, the term "nitrogen containing heterocycle" refers to a saturated or unsaturated monocyclic or bicyclic group having 2, 3, 4, 5, 6, 7, 8, 9, 10, 11, 12 or 13 carbon atoms and at least one nitrogen atom in the ring, which may further optionally comprise one or more (e.g., one, two, three or four) ring members selected from the group consisting of N, O, C=O, S, S=O and S(=O)₂. The nitrogen-containing heterocycle is connected with the rest of the molecule through a nitrogen atom. The nitrogen-containing heterocycle is preferably a saturated nitrogen-containing monocyclic ring. In particular, a 3- to 14-membered nitrogen-containing heterocycle is a group having 3-14 carbon atoms and heteroatoms (at least one of which is a nitrogen atom) in the ring, including but not limited to a 3-membered nitrogen-containing heterocycle (such as aziridinyl), a 4-membered nitrogen-containing heterocycle (such as azetidinyl), a 5-membered nitrogen-containing heterocycle (such as pyrrolyl, pyrrolidinyl (pyrrolidinyl ring), pyrrolinyl, pyrrolidonyl, imidazolyl, imidazolidinyl, imidazolinyl, pyrazolyl, pyrazolinyl), 6-membered nitrogen-containing heterocycle (such as piperidinyl (piperidinyl ring), morpholinyl, thiomorpholinyl, piperazinyl), 7-membered nitrogen-containing heterocycle, etc.

The term "substituted" means that one or more (e.g., one, two, three, or four) hydrogens on the designated atom is replaced with a selection from the indicated group, provided that the designated atom's normal valency under the existing circumstances is not exceeded, and that the substitution results in a stable compound. Combinations of substituents and/or variables are permissible only if such combinations result in stable compounds.

If a substituent is described as being "optionally substituted," the substituent may be either (1) not substituted, or (2) substituted. If a carbon of a substituent is described as being optionally substituted with one or more of a list of substituents, one or more of the hydrogens on the carbon (to the extent there are any) may separately and/or together be replaced with an independently selected optional substituent. If a nitrogen of a substituent is described as being optionally substituted with one or more of a list of substituents, one or more of the hydrogens on the nitrogen (to the extent there are any) may each be replaced with an independently selected optional substituent.

If substituents are described as being "independently selected" from a group, each substituent is selected independent of the other(s). Each substituent therefore may be identical to or different from the other substituent(s).

As used herein, the term "one or more" means one or more than one (e.g., 2, 3, 4, 5 or 10) as reasonable.

As used herein, unless specified, the point of attachment of a substituent can be from any suitable position of the substituent.

When a bond to a substituent is shown to cross a bond connecting two atoms in a ring, then such substituent may be bonded to any of the ring-forming atoms in that ring that are substitutable.

The present invention also includes all pharmaceutically acceptable isotopically labeled compounds, which are identical to those of the present invention except that one or more atoms are replaced by an atom having the same atomic number, but an atomic mass or mass number different from the atomic mass or mass number which predominates in nature. Examples of isotopes suitable for inclusion in the compound of the present invention include, but are not limited to, isotopes of hydrogen (such as deuterium (D, ²H), tritium (T, ³H)); carbon, such as ¹¹C, ¹³C, and ¹⁴C; chlorine, such as ³⁶Cl; fluorine, such as ¹⁸F; iodine, such as ¹²³I and ¹²⁵I; nitrogen, such as ¹³N and ¹⁵N; oxygen, such as ¹⁵O, ¹⁷O, and ¹⁸O; phosphorus, such as ³²P; and sulfur, such as ³⁵S. Certain isotopically labeled compounds of the present invention, for example those incorporating a radioactive isotope, are useful in drug and/or substrate tissue distribution studies (e.g., assays). The radioactive isotopes tritium, i.e., ³H, and carbon-14, i.e., ¹⁴C, are particularly useful for this purpose in view of their ease of incorporation and ready means of detection. Substitution with positron-emitting isotopes, such as ¹¹C, ¹⁸F, ¹⁵O and ¹³N, can be useful in positron emission tomography (PET) studies for examining substrate receptor occupancy. Isotopically labeled compounds of the present invention can generally be prepared by processes analogous to those described in the accompanying Schemes and/or in the Examples and Preparations, by using an appropriate isotopically labeled reagent in place of the non-labeled reagent previously employed. Pharmaceutically acceptable solvates in accordance with the invention include those wherein the solvent of crystallization may be isotopically substituted, e.g., D₂O, acetone-*d₆*, or DMSO-*d₆*.

The term "stereoisomer "refers to isomers with at least one asymmetric center. A compound having one or more (e.g., one, two, three or four) asymmetric centers can give rise to a racemic mixture, single enantiomer, diastereomer mixture and individual diastereomer. Certain individual molecules may exist as geometric isomers (cis/trans). Similarly, the compound of the present invention may exist as a mixture of two or more structurally different forms in rapid equilibrium (generally referred to as tautomer). Typical examples of a tautomer include a keto-enol tautomer, phenol-keto tautomer, nitroso-oxime tautomer, imine-enamine tautomer and the like. It is to be understood that all such isomers and mixtures thereof in any proportion (such as 60%, 65%, 70%, 75%, 80%, 85%, 90%, 95%, 96%, 97%, 98%, and 99%) are encompassed within the scope of the present invention.

The carbon-carbon bonds of the compound of the present invention may be depicted herein using a solid line ( 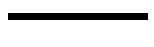 ), a solid wedge ( 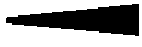 ), or a dotted wedge ( 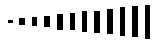 ). The use of a solid line to depict bonds to asymmetric carbon atoms is meant to indicate that all possible stereoisomers (e.g., specific enantiomers, racemic mixtures, *etc*.) at that carbon atom are included. The use of either a solid or dotted wedge to depict bonds to asymmetric carbon atoms is meant to indicate that the stereoisomer shown is present. When present in racemic compounds, solid and dotted wedges are used to define relative stereochemistry, rather than absolute stereochemistry. Unless stated otherwise, it is intended that the compound of the present invention can exist as stereoisomers, which include cis and trans isomers, optical isomers such as R and S enantiomers, diastereomers, geometric isomers, rotational isomers, conformational isomers, atropisomers, and mixtures thereof. The compound of the present invention may exhibit more than one type of isomerism, and consist of mixtures thereof (such as racemates and diastereomeric pairs).

The present invention includes all possible crystalline forms or polymorphs of the compound of the present invention, either as a single polymorph, or as a mixture of more than one polymorphs, in any ratio.

It also should be understood that, certain compounds of the present invention can be used for the treatment in a free form, or where appropriate, in a form of a pharmaceutically acceptable derivative. In the present invention, the pharmaceutically acceptable derivative includes, but is not limited to a pharmaceutically acceptable salt, ester, solvate, metabolite or prodrug, which can directly or indirectly provide the compound of the present invention or a metabolite or residue thereof after being administered to a patient in need thereof. Therefore, "the compound of the present invention" mentioned herein also means to encompass various derivative forms of the compound as mentioned above.

A pharmaceutically acceptable salt of the compound of the present invention includes an acid addition salt and a base addition salt thereof.

A suitable acid addition salt is formed from an acid which forms a pharmaceutically acceptable salt. Specific examples include aspartate, benzoate, bicarbonate/carbonate, bisulfate/sulfate, fumarate, gluceptate, gluconate, glucuronate, hexafluorophosphate, hydrobromide/bromide, hydroiodide/iodide, maleate, malonate, methylsulfate, naphthylate, nicotinate, nitrate, orotate, oxalate, palmitate and the like.

A suitable base addition salt is formed from a base which forms a pharmaceutically acceptable salt. Specific examples include aluminum, arginine, choline, diethylamine, lysine, magnesium, meglumine, potassium, and the like.

For a review on suitable salts, see "Hand book of Pharmaceutical Salts: Properties, Selection, and Use" by Stahl and Wermuth (Wiley-VCH, 2002). The method for preparing a pharmaceutically acceptable salt of the compound of the present invention is known to a person skilled in the art.

As used herein, the term "ester" refers to those derived from the compounds of the various formulae in the present application, which include physiologically-hydrolyzable esters (which may be hydrolyzed under physiological conditions to release the compounds of the present invention in the form of free acids or alcohols). The compound of the present invention itself may be an ester as well.

The compound of the present invention can exist as a solvate (preferably a hydrate), wherein the compound of the present invention contains a polar solvent, in particular water, methanol or ethanol for example, as a structural element of the crystal lattice of the compound. The amount of the polar solvent, in particular water, may exist in a stoichiometric or non-stoichiometric ratio.

The metabolite of the compound of the present invention, namely a substance formed *in vivo* upon administration of the compound of the present invention, is also included within the scope of the present invention. Such a product may result e.g., from the oxidation, reduction, hydrolysis, amidation, de-amidation, esterification, de-esterification, enzymolysis, and the like, of the administered compound. Accordingly, the present invention encompasses the metabolite of the compound of the present invention, including a compound produced by a method comprising contacting the compound of the present invention with a mammal for a period of time sufficient to result in a metabolic product thereof.

Also within the scope of the present invention is a prodrug of the compound of the invention, which is certain derivative of the compound of the invention that may have little or no pharmacological activity itself, but can, when administered into or onto the body, be converted into the compound of the invention having the desired activity, for example, by hydrolytic cleavage. In general, such prodrug will be a functional derivative of the compound which is readily converted *in vivo* into the compound with desired therapeutic activity. Further information on the use of the prodrug may be found in "Pro-drugs as Novel Delivery Systems, Vol. 14, ACS Symposium Series (T. Higuchi and V. Stella) and "Bioreversible Carriers in Drug Design," Pergamon Press, 1987 (edited by E. B. Roche, American Pharmaceutical Association). The prodrug in accordance with the invention can, for example, be produced by replacing appropriate functionalities present in the compound of the present invention with certain moieties known to those skilled in the art as "pro-moieties" as described, for example, in "Design of Prodrugs" by H. Bundgaard (Elsevier, 1985).

The present invention further encompasses the compound of the present invention having a protecting group. During any of the processes for preparation of the compound of the present invention, it may be necessary and/or desirable to protect sensitive or reactive groups on any of the molecules concerned, thereby resulting in the chemically protected form of the compound of the present invention. This may be achieved by means of conventional protecting groups, e.g., those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991, which is incorporated herein by reference. The protecting groups may be removed at a convenient subsequent stage using methods known from the art.

As used herein, the term "about" refers to a range within ±10%, preferably within ±5%, and more preferably within ±2% of the specified value.

### Compound

In some embodiments, the present invention provides a compound or a pharmaceutically acceptable salt, ester, stereoisomer, tautomer, polymorph, solvate, metabolite, isotopically labeled compound or prodrug thereof, wherein the compound has the structure of Formula (I): 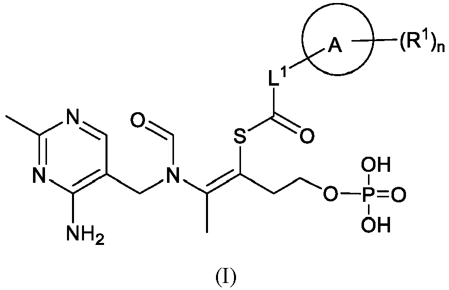 wherein:
ring A is a C₆₋₁₀ aromatic ring or 5- to 14-membered heteroaromatic ring;
L' is a direct bond or -R²-C₁₋₆ alkylene-;
R² is -O-, -NH-, -S-, -S(=O)- or -S(=O)₂-;
R¹, at each occurrence, is each independently selected from the group consisting of halogen, hydroxyl, oxo, amino, cyano, nitro, C₁₋₆ alkyl, C₂₋₆ alkenyl, C₂₋₆ alkynyl, C₃₋₆ cyclic hydrocarbyl, 3- to 10-membered heterocyclyl, C₆₋₁₀ aryl, 5- to 14-membered heteroaryl, C₆₋₁₂ aralkyl, =N-OR³, -C(=NH)NH₂, -C(=O)R³, -OC(=O)R³, -C(=O)OR³, -OR³, -SR³, -S(=O)R³, -S(=O)₂R³, -S(=O)₂NR³R⁴, -NR³R⁴, -C(=O)NR³R⁴, -NR³-C(=O)R⁴, -NR³-C(=O)OR⁴, -NR³-S(=O)₂-R⁴, -NR³-C(=O)-NR³R⁴, -C₁₋₆ alkylene-NR³R⁴, -O-C₁₋₆ alkylene-NR³R⁴ and -C₁₋₆ alkylene-O-C₁₋₆ alkyl; or when n is greater than 1, two R¹ together with the group to which they are attached form a C₃₋₆ hydrocarbon ring, 3- to 10-membered heterocycle, C₆₋₁₀ aromatic ring or 5- to 14-membered heteroaromatic ring;
R³ and R⁴, at each occurrence, are each independently selected from the group consisting of H, C₁₋₆ alkyl, C₃₋₁₀ cyclic hydrocarbyl, 3- to 10-membered heterocyclyl, C₆₋₁₀ aryl, 5- to 14-membered heteroaryl and C₆₋₁₂ aralkyl;
the above alkyl, alkylene, alkenyl, alkynyl, cyclic hydrocarbyl, hydrocarbon ring, heterocyclyl, heterocycle, aryl, aromatic ring, heteroaryl, heteroaromatic ring and aralkyl, at each occurrence, are each optionally substituted with one or more substituents independently selected from the group consisting of: halogen, hydroxyl, oxo, amino, cyano, nitro, C₁₋₆ alkyl, C₂₋₆ alkenyl, C₂₋₆ alkynyl, C₃₋₆ cyclic hydrocarbyl, 3- to 10-membered heterocyclyl, C₆₋₁₀ aryl, 5- to 14-membered heteroaryl, C₆₋₁₂ aralkyl, =N-OR⁵, -C(=NH)NH₂, -C(=O)R⁵, -OC(=O)R⁵, -C(=O)OR⁵, -OR⁵, -SR⁵, -S(=O)R⁵, -S(=O)₂R⁵, -S(=O)₂NR⁵R⁶, -NR⁵R⁶, -C(=O)NR⁵R⁶, -NR⁵-C(=O)R⁶, -NR⁵-C(=O)OR⁶, -NR⁵-S(=O)₂-R⁶, -NR⁵-C(=O)-NR⁵R⁶, -C₁₋₆ alkylene-NR⁵R⁶, -O-C₁₋₆ alkylene-NR⁵R⁶ and -C₁₋₆ alkylene-O-C₁₋₆ alkyl, the alkyl, alkenyl, alkynyl, cyclic hydrocarbyl, heterocyclyl, aryl, heteroaryl and aralkyl are further optionally substituted with one or more substituents independently selected from the group consisting of: halogen, hydroxyl, oxo, amino, cyano, nitro, C₁₋₆ alkyl, C₃₋₆ cyclic hydrocarbyl, 3- to 10-membered heterocyclyl, C₆₋₁₀ aryl, 5- to 14-membered heteroaryl and C₆₋₁₂ aralkyl;
R⁵ and R⁶, at each occurrence, are each independently selected from the group consisting of H, C₁₋₆ alkyl, C₃₋₁₀ cyclic hydrocarbyl, 3- to 10-membered heterocyclyl, C₆₋₁₀ aryl, 5- to 14-membered heteroaryl and C₆₋₁₂ aralkyl; and
n is an integer of 0, 1, 2, 3 or 4;
provided that, when L' is a direct bond, ring A is not a benzene ring.

In some embodiments, the present invention provides the compound of Formula (I) or a pharmaceutically acceptable salt, ester, stereoisomer, tautomer, polymorph, solvate, metabolite, isotopically labeled compound or prodrug thereof, wherein ring A is a benzene ring or 5- to 6-membered heteroaromatic ring.

In some embodiments, the present invention provides the compound of Formula (I) or a pharmaceutically acceptable salt, ester, stereoisomer, tautomer, polymorph, solvate, metabolite, isotopically labeled compound or prodrug thereof, wherein ring A is a benzene ring, pyrrole ring, furan ring, thiophene ring or pyridine ring.

In some embodiments, the present invention provides the compound of Formula (I) or a pharmaceutically acceptable salt, ester, stereoisomer, tautomer, polymorph, solvate, metabolite, isotopically labeled compound or prodrug thereof, wherein L' is a direct bond or -O-C₁₋₆ alkylene-.

In some embodiments, the present invention provides the compound of Formula (I) or a pharmaceutically acceptable salt, ester, stereoisomer, tautomer, polymorph, solvate, metabolite, isotopically labeled compound or prodrug thereof, wherein L' is a direct bond, -O-CH₂- or -O-CH₂CH₂-.

In some embodiments, when L' is a direct bond and n is 0, ring Ais not an unsubstituted furan ring or unsubstituted thiophene ring.

In some embodiments, the present invention provides the compound of Formula (I) or a pharmaceutically acceptable salt, ester, stereoisomer, tautomer, polymorph, solvate, metabolite, isotopically labeled compound or prodrug thereof, wherein R¹ is halogen, C₁₋₆ alkyl, C₆₋₁₀ aryl optionally substituted with halogen or -C₁₋₆ alkylene-O-C₁₋₆ alkyl; or two R¹ together with the group to which they are attached form a C₆₋₁₀ aromatic ring, which is optionally further substituted with -OR⁵.

In some embodiments, the present invention provides the compound of Formula (I) or a pharmaceutically acceptable salt, ester, stereoisomer, tautomer, polymorph, solvate, metabolite, isotopically labeled compound or prodrug thereof, wherein R¹ is -Cl, methyl, phenyl optionally substituted with F or -CH₂-O-CH₃; or two R¹ together with the group to which they are attached form a benzene ring, which is optionally further substituted with methoxy.

In some embodiments, the present invention provides the compound of Formula (I) or a pharmaceutically acceptable salt, ester, stereoisomer, tautomer, polymorph, solvate, metabolite, isotopically labeled compound or prodrug thereof, wherein

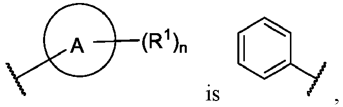

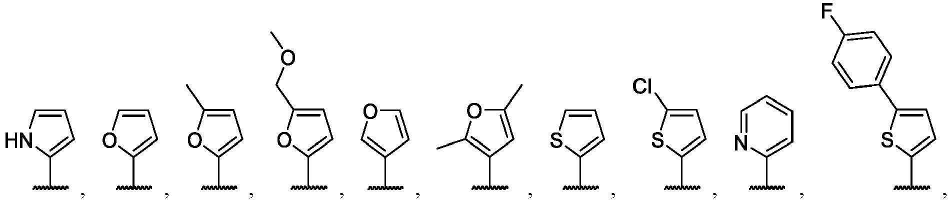

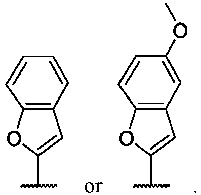

The compound obtained by any combination of the various embodiments is encompassed by the invention.

In some embodiments, the compound of Formula (I) is not the following compounds:

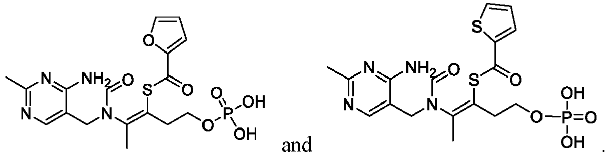

In some embodiments, the present invention provides a compound or a pharmaceutically acceptable salt, ester, stereoisomer, tautomer, polymorph, solvate, metabolite, isotopically labeled compound or prodrug thereof, wherein the compound is:

| **No.** | **Structural Formula** | **Compound Name** |
|---|---|---|
| 1 | 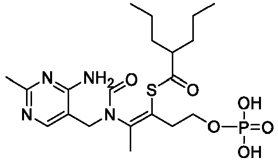 | (Z)-S-(2-(N-((4-amino-2-methylpyrimidin-5-yl)methyl)forma mido)-5-(phosphonooxy)pent-2-en-3-yl) 2-propylpentanethioate |
| 2 | 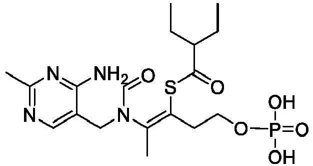 | (Z)-S-(2-(N-((4-amino-2-methylpyrimidin-5-yl)methyl)forma mido)-5 -(phosphonooxy)pent-2-en-3-yl)2-ethylbutanethioate |
| 5 | 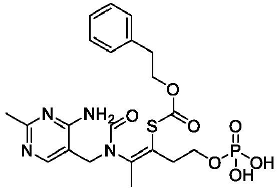 | (Z)-S-(2-(N-((4-amino-2-methylpyrimidin-5-yl)methyl)forma mido)-5-(phosphonooxy)pent-2-en-3-yl)O-phenethyl carbonothioate |
| 6 | 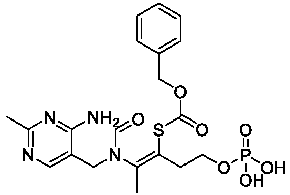 | (Z)-S-(2-(N-((4-amino-2-methylpyrimidin-5-yl)methyl)forma mido)-5-(phosphonooxy)pent-2-en-3-yl) O-benzylcarbonothioate |
| 7 | 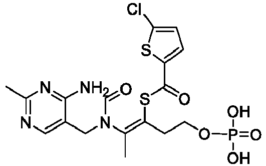 | (Z)-S-(2-(N-((4-amino-2-methylpyrimidin-5-yl)methyl)forma mido)-5-(phosphonooxy)pent-2-en-3-yl) 5-chlorothiophene-2-carbothioate |
| 8 | 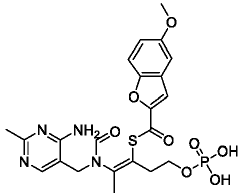 | (Z)-S-(2-(N-((4-amino-2-methylpyrimidin-5-yl)methyl)forma mido)-5-(phosphonooxy)pent-2-en-3-yl) 5-methoxybenzofuran-2-carbothioate |
| 9 | 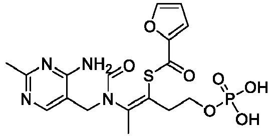 | (Z)-S-(2-(N-((4-amino-2-methylpyrimidin-5-yl)methyl)forma mido)-5 -(phosphonooxy)pent-2-en-3 -yl) furan-2-carbothioate |
| 10 | 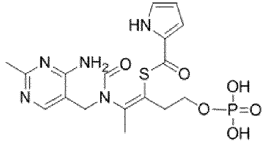 | (Z)-S-(2-(N-((4-amino-2-methylpyrimidin-5-yl)methyl)forma mido)-5-(phosphonooxy)pent-2-en-3-yl) 1H-pyrrole-2-carbothioate |
| 11 | 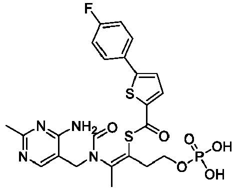 | (Z)-S-(2-(N-((4-amino-2-methylpyrimidin-5-yl)methyl)forma mido)-5-(phosphonooxy)pent-2-en-3-yl) 5-(4-fluorophenyl)thiophene-2-carbothioate |
| 12 | 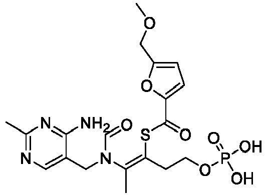 | (Z)-S-(2-(N-((4-amino-2-methylpyrimidin-5-yl)methyl)forma mido)-5 -(phosphonooxy)pent-2-en-3-yl) 5-(methoxymethyl)furan-2-carbothioate |
| 13 | 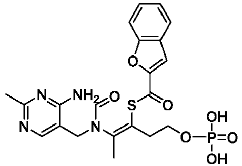 | (Z)-S-(2-(N-((4-amino-2-methylpyrimidin-5-yl)methyl)forma mido)-5 -(phosphonooxy)pent-2-en-3-yl) benzofuran-2-carbothioate |
| 14 | 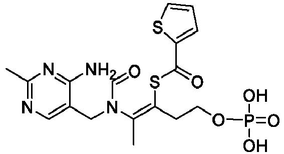 | (Z)-S-(2-(N-((4-amino-2-methylpyrimidin-5-yl)methyl)forma mido)-5-(phosphonooxy)pent-2-en-3-yl) thiophene-2-carbothioate |
| 15 | 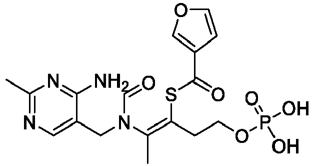 | (Z)-S-(2-(N-((4-amino-2-methylpyrimidin-5-yl)methyl)forma mido)-5 -(phosphonooxy)pent-2-en-3-yl)furan-3-carbothioate |
| 16 | 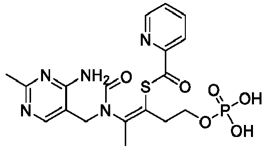 | (Z)-S-(2-(N-((4-amino-2-methylpyrimidin-5-yl)methyl)forma mido)-5-(phosphonooxy)pent-2-en-3-yl) pyridine-2-carbothioate |
| 17 | 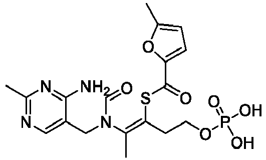 | (Z)-S-(2-(N-((4-amino-2-methylpyrimidin-5-yl)methyl)forma mido)-5-(phosphonooxy)pent-2-en-3-yl) 5-methylfuran-2-carbothioate |
| 18 | 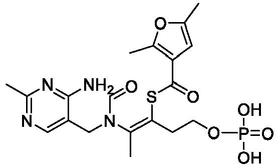 | (Z)-S-(2-(N-((4-amino-2-methylpyrimidin-5-yl)methyl)forma mido)-5-(phosphonooxy)pent-2-en-3-yl) 2,5-dimethylfuran-3-carbothioate. |

### Preparation Method

In some embodiments, the present invention provides a method for preparing the compound of Formula (I): 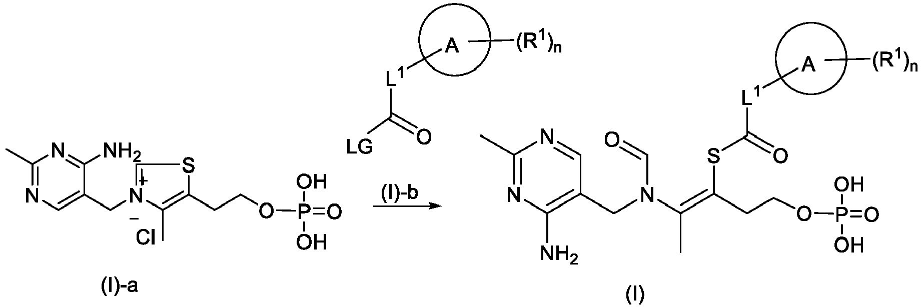 wherein:
LG is a leaving group, preferably halogen, most preferably chlorine;
the remaining groups are as defined above;
the method comprises reacting a compound of formula (I)-a with a compound of formula (I)-b to obtain the compound of formula (I).

The reaction is preferably carried out in the presence of a base, such as an inorganic base (e.g., sodium hydroxide) or an organic base; preferably, the compound of formula (I)-a is firstly mixed with the base, and then the compound of formula (I)-b is added to the resulting mixture. The reaction solvent is preferably water, dichloromethane, tetrahydrofuran or a mixture thereof (e.g., a mixture of water and tetrahydrofuran). The reaction temperature is preferably 0-50°C, e.g., 25°C.

### Pharmaceutical composition and therapeutic method

In some embodiments, the present invention provides a pharmaceutical composition comprising a prophylactically or therapeutically effective amount of the compound of the present invention or a pharmaceutically acceptable salt, ester, stereoisomer, tautomer, polymorph, solvate, metabolite, isotopically labeled compound or prodrug thereof and one or more pharmaceutically acceptable carriers.

In some embodiments, the present invention provides use of the compound of the present invention or a pharmaceutically acceptable salt, ester, stereoisomer, tautomer, polymorph, solvate, metabolite, isotopically labeled compound or prodrug thereof or the pharmaceutical composition of the present invention in the manufacture of a medicament for the prophylaxis or treatment of a neurodegenerative disease or the alleviation of a symptom of the neurodegenerative disease.

In some embodiments, the present invention provides the compound of the present invention or a pharmaceutically acceptable salt, ester, stereoisomer, tautomer, polymorph, solvate, metabolite, isotopically labeled compound or prodrug thereof or the pharmaceutical composition of the present invention for use in the prophylaxis or treatment of a neurodegenerative disease or the alleviation of a symptom of the neurodegenerative disease.

In some embodiments, the present invention provides a method for the prophylaxis or treatment of a neurodegenerative disease or the alleviation of a symptom of the neurodegenerative disease, wherein the method comprises administering to a subject in need thereof an effective amount of the compound of the present invention or a pharmaceutically acceptable salt, ester, stereoisomer, tautomer, polymorph, solvate, metabolite, isotopically labeled compound or prodrug thereof or the pharmaceutical composition of the present invention.

In some embodiments, the neurodegenerative disease is selected from the group consisting of Alzheimer's disease, Creutzfeldt-Jakob disease, Huntington's disease, multiple sclerosis, Guillain-Barre syndrome, Parkinson's disease, Lou Gehrig's disease, paralytic dementia caused by gradual nerve cell death and diseases caused by progressive incontinentia; preferably Alzheimer's disease.

The term "pharmaceutically acceptable carrier" in the present invention refers to a diluent, auxiliary material, excipient, or vehicle with which a therapeutic is administered, and it is, within the scope of sound medical judgment, suitable for contact with the tissues of human beings and animals without excessive toxicity, irritation, allergic response, or other problem or complication, commensurate with a reasonable benefit/risk ratio.

The pharmaceutically acceptable carrier which can be employed in the pharmaceutical composition of the present invention includes, but is not limited to sterile liquids, such as water and oils, including those of petroleum, animal, vegetable or synthetic origin, such as peanut oil, soybean oil, mineral oil, sesame oil and the like. Water is an exemplary carrier when the pharmaceutical composition is administered intravenously. Physiological salines as well as aqueous dextrose and glycerol solutions can also be employed as liquid carriers, particularly for injectable solutions. Suitable pharmaceutical excipients include starch, glucose, lactose, sucrose, gelatin, maltose, chalk, silica gel, sodium stearate, glycerol monostearate, talc, sodium chloride, dried skim milk, glycerol, propylene glycol, water, ethanol and the like. The pharmaceutical composition, if desired, can also contain minor amounts of wetting or emulsifying agents, or pH buffering agents. Oral formulations can include standard carriers such as pharmaceutical grades of mannitol, lactose, starch, magnesium stearate, sodium saccharine, cellulose, magnesium carbonate, *etc.* Examples of suitable pharmaceutical carriers are described in e.g. Remington's Pharmaceutical Sciences (1990).

The pharmaceutical composition of the present invention can act systemically and/or topically. To this end, it can be administered through a suitable route, such as through injection, (intravenous, intraarterial, subcutaneous, intraperitoneal, intramuscular injection, including dripping), or transdermal administration, or administered via oral, buccal, nasal, transmucosal, topical, as an ophthalmic formulation, or via inhalation.

For these routes of administration, the pharmaceutical composition of the present invention can be administered in a suitable dosage form.

Such dosage forms include, but are not limited to tablets, capsules, lozenges, hard candies, powders, sprays, creams, salves, suppositories, gels, pastes, lotions, ointments, aqueous suspensions, injectable solutions, elixirs, and syrups.

As used herein, the term "effective amount" refers to the amount of a compound being administered which will relieve to some extent one or more of the symptoms of the disorder being treated.

Dosage regimens may be adjusted to provide the optimum desired response. For example, a single bolus may be administered, several divided doses may be administered over time, or the dose may be proportionally reduced or increased as indicated by the exigencies of the therapeutic situation. It is to be noted that dosage values may vary with the type and severity of the condition to be alleviated, and may include single or multiple doses. It is to be further understood that for any particular subject, specific dosage regimens should be adjusted over time according to the individual need and the professional judgment of the person administering or supervising the administration of the composition.

The amount of the compound of the present invention administered will be dependent on the subject being treated, the severity of the disorder or condition, the rate of administration, the disposition of the compound and the discretion of the prescribing physician. Generally, an effective dosage is in the range of about 0.0001 to about 50 mg per kg body weight per day, for example about 0.01 to about 10 mg/kg/day, in single or divided doses. For a 70 kg human, this would amount to about 0.007 mg to about 3500 mg/day, for example about 0.7 mg to about 700 mg/day. In some instances, dosage levels below the lower limit of the aforesaid range may be more than adequate, while in other cases, still larger doses may be employed without causing any harmful side effect, provided that such larger doses are first divided into several small doses for administration throughout the day.

The content or dosage of the compound of the present invention in the pharmaceutical composition is about 0.01 mg to about 1000 mg, suitably 0.1-500 mg, preferably 0.5-300 mg, more preferably 1-150 mg, particularly preferably 1-50 mg, e.g., 1.5 mg, 2 mg, 4 mg, 10 mg, 25 mg, *etc.*

Unless otherwise indicated, the term "treating" or "treatment", as used herein, means reversing, alleviating, inhibiting the progress of, or preventing the disorder or condition to which such term applies, or one or more symptoms of such disorder or condition.

As used herein, the term "subject" includes a human or non-human animal. An exemplary human subject includes a human subject having a disease (such as one described herein) (referred to as a patient), or a normal subject. The term "non-human animal" as used herein includes all vertebrates, such as non-mammals (e.g. birds, amphibians, reptiles) and mammals, such as non-human primates, livestock and/or domesticated animals (such as sheep, dog, cat, cow, pig and the like).

In some embodiments, the pharmaceutical composition of the present invention can further comprise one or more additional therapeutic agents or prophylactic agents.

### Examples

In order to make the object and technical solutions of the present invention more clear, the embodiments of the present invention will be described in detail with reference to the following examples. However, a person skilled in the art will understand that the following examples are only for illustrating the invention and should not be construed as limiting the scope of the invention. Where specific conditions are not specified in the examples, they shall be carried out according to the conventional conditions or the conditions suggested by the manufacturer. If the manufacturer is not indicated on the reagents or instruments used, they are all conventional products that can be purchased in the market.

The structure of the compound was determined by nuclear magnetic resonance (¹H NMR) or mass spectrum (MS). The NMR was determined by Bruker AVANCE-500 or Varian-400MHz NMR. The solvents employed were deuterated dimethyl sulfoxide (DMSO-*d₆*), deuterated methanol (CD₃OD), deuterated water (D₂O), *etc.* The internal standard was tetramethylsilane (TMS), and the chemical shift (δ) was given in parts per million (ppm).

The instrument for MS measurement was Agilent (ESI) mass spectrometer (manufacturer: Agilent, model: Agilent 6110).

### Method for preparative high performance liquid chromatography separation:

Instrument model: Elite P3500, chromatographic column: welch Ultimate XB-C18 (30×250 mm, 10 µm); chromatographic column temperature, 25°C; flow rate: 42 mL/min; detection wavelength: 254 nm; elution gradient: (0 min: 10% A, 90% B; 25 min: 90% A, 10% B; 35 min: 90% A, 10% B; 38 min: 10% A, 90% B; 40 min: 10% A, 90% B); mobile phase: A: methanol, B: a 0.05% aqueous solution of formic acid.

The compounds synthesized in the following examples are subject to the compounds represented by the molecular formula, and the compound names are generated by ChemBioDraw software.

### Example 1: synthesis of (Z)-S-(2-(N-((4-amino-2-methylpyrimidin-5-yl)methyl)formamido)-5-(phosphonooxy)pent-2-en-3-yl) 2-propylpentanethioate (compound 1)

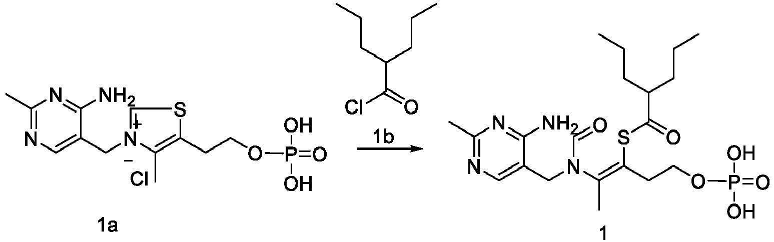

Thiamine monophosphate chloride (1a) (content 83%, 20 g, 0.04 mol, 1.0 eq.) was dissolved in water (40 mL), and it was dissolved by stirring. A solution of sodium hydroxide (30%) was dropwise added to adjust the pH to 10.5-11.0, and the mixture was stirred for 30 minutes. The system temperature was maintained at 25°C, and 2-propylpentanoyl chloride (1b) (0.07 mol, 1.75 eq.) diluted with tetrahydrofuran (20 mL) was dropwise added within 20 minutes. The mixture was stirred for 10 minutes, the pH of the aqueous phase was adjusted to 1.5. The system became turbid, and ethyl acetate (100 mL) was slowly added dropwise. White solid was precipitated, filtered and dried, and then dissolved in water (100mL). The mixture was added with NaHCOs (1.0 eq.), stirred to generate a few bubbles, concentrated to dryness, added with DCM, stirred and dissolved, filtered to remove salt, and the filtrate was concentrated to obtain title compound 1 (white solid).
MS m/z (ESI): 489 [M+1]
¹H NMR (400 MHz, DMSO-*d₆*): δ 7.90 (s, 1H), 7.74 (s, 1H), 4.42 (s, 2H), 3.74-3.76 (m, 2H), 2.58 (s, 2H), 2.42-2.43 (m, 1H), 2.38 (s, 3H), 2.10 (s, 3H), 1.41-1.44 (m, 2H), 1.30-1.35 (m, 2H), 1.28-1.33 (m, 4H), 0.80-0.83 (t, 6H).

### Example 2: synthesis of (Z)-S-(2-(N-((4-amino-2-methylpyrimidin-5-yl)methyl)formamido)-5-(phosphonooxy)pent-2-en-3-yl) 2-ethylbutanethioate (compound 2)

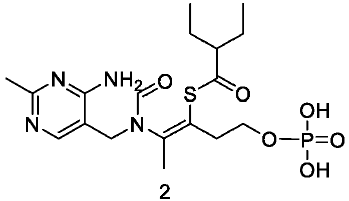

The title compound 2 (white solid) was prepared by the same synthetic route as in Example 1 except that 1b in Example 1 was replaced by 2-ethylbutanoyl chloride.
MS m/z (ESI): 461 [M+1]
¹H NMR (400MHz, DMSO-*d₆*) δ 9.18 (br, 1H), 8.19 (s, 1H), 8.15 (br, 1H), 7.84 (s, 1H), 4.48 (s, 2H), 3.89 (q, 2H), 2.63 (t, 2H), 2.48 (s, 3H), 2.34-2.27 (m, 1H), 2.14 (s, 3H), 1.51-1.37 (m, 4H), 0.80 (t, 6H).

### Example 5: synthesis of (Z)-S-(2-(N-((4-amino-2-methylpyrimidin-5-yl)methyl)formamido)-5-(phosphonooxy)pent-2-en-3-yl) O-phenethyl carbonothioate (compound 5)

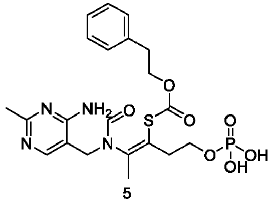

The title compound 5 (off-white solid) was prepared by the same synthetic route as in Example 1 except that 1b in Example 1 was replaced by phenethyl chloroformate.
MS m/z (ESI): 511.1 [M+1]
¹H NMR (500 MHz, DMSO-*d₆*) δ 7.84 (s, 2H), 7.29-7.21 (m, 5H), 6.73 (s, 2H), 4.25 (m, 4H), 3.65 (m, 4H), 2.87 (t, 2H), 2.25 (s, 3H), 2.07 (s, 3H).

### Example 6: synthesis of (Z)-S-(2-(N-((4-amino-2-methylpyrimidin-5-yl)methyl)formamido)-5-(phosphonooxy)pent-2-en-3-yl) O-benzylcarbonothioate (compound 6)

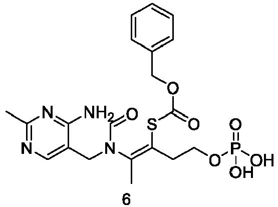

The title compound 6 (off-white solid) was prepared by the same synthetic route as in Example 1 except that 1b in Example 1 was replaced by benzyl chloroformate.
MS m/z (ESI): 497.1 [M+1]
¹H NMR (500 MHz, DMSO-*d₆*) δ 7.84 (m, 2H), 7.37 (m, 5H), 6.75 (s, 2H), 5.04 (m, 2H), 4.37 (m, 2H), 3.37 (m, 2H) 2.59 (m, 2H), 2.26 (s, 3H), 2.08 (s, 3H).

### Example 7: synthesis of (Z)-S-(2-(N-((4-amino-2-methylpyrimidin-5-yl)methyl)formamido)-5-(phosphonooxy)pent-2-en-3-yl) 5-chlorothiophene-2-carbothioate (compound 7)

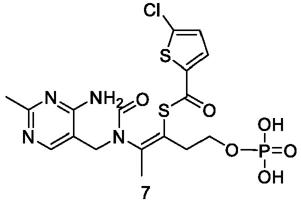

The title compound 7 (white solid) was prepared by the same synthetic route as in Example 1 except that 1b in Example 1 was replaced by 5-chlorothiophene-2-carbonyl chloride.
MS m/z (ESI): 507 [M+1]
¹H NMR (500 MHz, DMSO-*d₆*) δ 7.95 (s, 1H), 7.92 (s, 1H), 7.61 (d, 1H), 7.31 (d, 1H), 4.49 (s, 2H), 3.86 (t, 2H), 2.70 (t, 2H), 2.33 (s, 3H), 2.20 (s, 3H).

### Example 8: synthesis of (Z)-S-(2-(N-((4-amino-2-methylpyrimidin-5-yl)methyl)formamido)-5-(phosphonooxy)pent-2-en-3-yl) 5-methoxybenzofuran-2-carbothioate (compound 8)

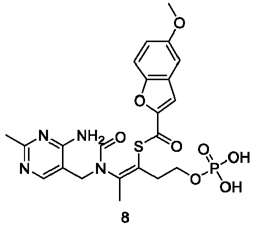

The title compound 8 (off-white solid) was prepared by the same synthetic route as in Example 1 except that 1b in Example 1 was replaced by 5-methoxybenzofuran-2-carbonyl chloride.
MS m/z (ESI): 537 [M+1]
¹H NMR (500 MHz, DMSO-*d₆*) δ 7.95 (s, 2H), 7.63 (s, 2H), 7.28 (s, 1H), 7.18 (s, 1H), 4.47 (s, 2H), 3.86 (q, 2H), 3.82 (s, 3H), 2.71 (t, 2H), 2.28 (s, 3H), 2.20 (s, 3H).

### Example 9: synthesis of (Z)-S-(2-(N-((4-amino-2-methylpyrimidin-5-yl)methyl)formamido)-5-(phosphonooxy)pent-2-en-3-yl) furan-2-carbothioate (compound 9)

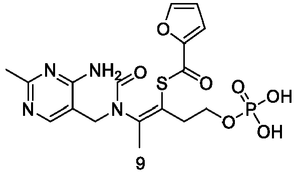

The title compound 9 (off-white solid) was prepared by the same synthetic route as in Example 1 except that 1b in Example 1 was replaced by furan-2-carbonyl chloride.
MS m/z (ESI): 456.9 [M+1]
¹H NMR (500 MHz, DMSO-*d₆*) δ 8.02 (s, 1H), 7.85 (s, 1H), 7.83 (s, 1H), 7.27 (d, 1H), 6.75 (t, 1H), 4.45 (s,2H), 3.85 (q, 2H), 2.70 (t, 2H), 2.30 (s, 3H), 2.17 (s, 3H).

### Example 10: synthesis of (Z)-S-(2-(N-((4-amino-2-methylpyrimidin-5-yl)methyl)formamido)-5-(phosphonooxy)pent-2-en-3-yl) 1H-pyrrole-2-carbothioate (compound 10)

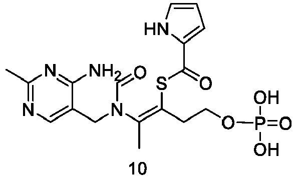

The title compound 10 (off-white solid) was prepared by the same synthetic route as in Example 1 except that 1b in Example 1 was replaced by 1H-pyrrole-2-carbonyl chloride.
MS m/z (ESI): 456.0 [M+1]
¹H NMR (500 MHz, DMSO-*d₆*) δ 12.12 (s, 1H), 7.88 (s, 1H), 7.86 (s, 1H), 7.14 (s, 1H), 6.77 (d, 1H), 6.20 (m, 1H), 4.45 (s, 2H), 3.85 (q, 2H), 2.70 (t, 2H), 2.36 (s, 3H), 2.15 (s, 3H).

### Example 11: synthesis of (Z)-S-(2-(N-((4-amino-2-methylpyrimidin-5-yl)methyl)formamido)-5-(phosphonooxy)pent-2-en-3-yl) 5-(4-fluorophenyl)thiophene-2-carbothioate (compound 11)

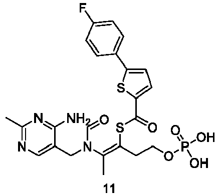

The title compound 11 (off-white solid) was prepared by the same synthetic route as in Example 1 except that 1b in Example 1 was replaced by 5-(4-fluorophenyl)thiophene-2-carbonyl chloride.
MS m/z (ESI): 567 [M+1]
¹H NMR (500 MHz, DMSO-*d₆*) δ 7.92 (s, 2H), 7.84 (dd, 2H), 7.70 (d, 1H), 7.62 (d, 1H), 7.35 (dd, 2H), 4.48 (s, 2H), 3.88 (q, 2H), 2.72 (t, 2H), 2.31 (s, 3H), 2.19 (s, 3H).

### Example 12: synthesis of (Z)-S-(2-(N-((4-amino-2-methylpyrimidin-5-yl)methyl)formamido)-5-(phosphonooxy)pent-2-en-3-yl) 5-(methoxymethyl)furan-2-carbothioate (compound 12)

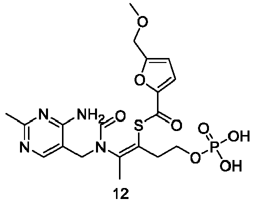

The title compound 12 (off-white solid) was prepared by the same synthetic route as in Example 1 except that 1b in Example 1 was replaced by 5-(methoxymethyl)furan-2-carbonyl chloride.
MS m/z (ESI): 501.5 [M+1]
¹H NMR (500 MHz, DMSO-*d₆*) δ 7.88 (s, 2H), 7.40 (s, 2H), 7.24 (s, 1H), 6.69 (s, 1H), 4.44 (s, 4H), 3.86 (q, 2H), 3.30 (s, 3H), 2.72 (t, 2H), 2.32 (s, 3H), 2.16 (s, 3H).

### Example 13: synthesis of (Z)-S-(2-(N-((4-amino-2-methylpyrimidin-5-yl)methyl)formamido)-5-(phosphonooxy)pent-2-en-3-yl) benzofuran-2-carbothioate (compound 13)

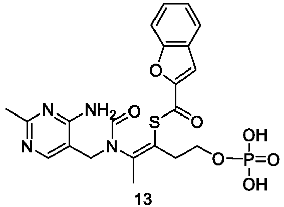

The title compound 13 (white solid) was prepared by the same synthetic route as in Example 1 except that 1b in Example 1 was replaced by benzofuran-2-carbonyl chloride.
MS m/z (ESI): 507 [M+1]
¹H NMR (500 MHz, DMSO-*d₆*) δ 7.98 (d, 2H), 7.83 (d, 1H), 7.73 (s, 2H), 7.58 (t, 1H), 7.40 (t, 1H), 4.49 (s, 2H), 3.89 (d, 2H), 2.72 (s, 2H), 2.29 (s, 3H), 2.21 (s, 3H).

### Example 14: synthesis of (Z)-S-(2-(N-((4-amino-2-methylpyrimidin-5-yl)methyl)formamido)-5-(phosphonooxy)pent-2-en-3-yl) thiophene-2-carbothioate (compound 14)

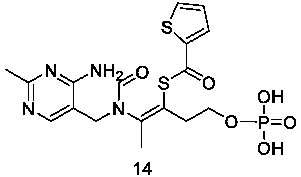

The title compound 14 (white solid) was prepared by the same synthetic route as in Example 1 except that 1b in Example 1 was replaced by thiophene-2-carbonyl chloride.
MS m/z (ESI): 473 [M+1]
¹H NMR (500 MHz, DMSO-*d₆*) δ 8.08 (d, 1H), 7.92 (s, 1H), 7.90 (s, 1H), 7.71 (d, 1H), 7.24 (t, 1H), 4.47 (s,2H), 3.85 (q, 2H), 2.70 (t, 2H), 2.31 (s, 3H), 2.18 (s, 3H).

### Example 15: synthesis of (Z)-S-(2-(N-((4-amino-2-methylpyrimidin-5-yl)methyl)formamido)-5-(phosphonooxy)pent-2-en-3-yl) furan-3-carbothioate (compound 15)

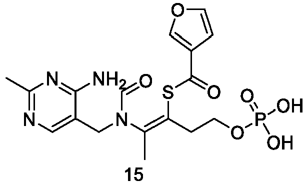

The title compound 15 (off-white solid) was prepared by the same synthetic route as in Example 1 except that 1b in Example 1 was replaced by furan-3-carbonyl chloride.
MS m/z (ESI): 457 [M+1]
¹H NMR (500 MHz, DMSO-*d₆*) δ 8.39 (s, 1H), 7.96 (s, 1H), 7.88 (s, 1H), 7.86 (s, 1H), 6.68 (d, 1H), 4.49 (s, 2H), 3.86 (q, 2H), 2.69 (t, 2H), 2.34 (s, 3H), 2.17 (s, 3H).

### Example 16: synthesis of (Z)-S-(2-(N-((4-amino-2-methylpyrimidin-5-yl)methyl)formamido)-5-(phosphonooxy)pent-2-en-3-yl) pyridine-2-carbothioate (compound 16)

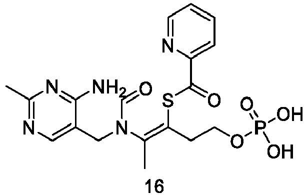

The title compound 16 (off-white solid) was prepared by the same synthetic route as in Example 1 except that 1b in Example 1 was replaced by 2-pyridylcarbonylchloride.
MS m/z (ESI): 468.1 [M+1]
¹H NMR (500 MHz, DMSO-d₆) δ 8.67 (d, 1H), 8.03 (t, 1H), 7.90 (s, 2H), 7.78 (d, 1H), 7.73 (t, 1H), 7.28 (s, 2H), 4.43 (s,2H), 3.86 (q, 2H), 2.70 (t, 2H), 2.25 (s, 3H), 2.16 (s, 3H).

### Example 17: synthesis of (Z)-S-(2-(N-((4-amino-2-methylpyrimidin-5-yl)methyl)formamido)-5-(phosphonooxy)pent-2-en-3-yl) 5-methylfuran-2-carbothioate (compound 17)

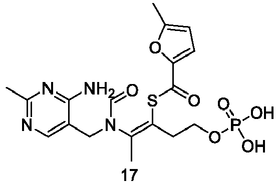

The title compound 17 (off-white solid) was prepared by the same synthetic route as in Example 1 except that 1b in Example 1 was replaced by 5-methylfuran-2-carbonyl chloride.
MS m/z (ESI): 471 [M+1]
¹H NMR (500 MHz, DMSO-d₆) δ 7.86 (s, 2H), 7.19 (d, 1H), 7.40 (d, 1H), 4.44 (s, 2H), 3.86 (q, 2H), 2.67 (t, 2H), 2.37 (s, 3H), 2.32 (s, 3H), 2.15 (s, 3H).

### Example 18: synthesis of (Z)-S-(2-(N-((4-amino-2-methylpyrimidin-5-yl)methyl)formamido)-5-(phosphonooxy)pent-2-en-3-yl) 2,5-dimethylfuran-3-carbothioate (compound 18)

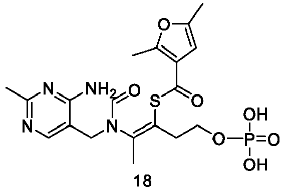

The title compound 18 (off-white solid) was prepared by the same synthetic route as in Example 1 except that 1b in Example 1 was replaced by 2,5-dimethylfuran-3-carbonyl chloride.
MS m/z (ESI): 485 [M+1]
¹H NMR (500 MHz, DMSO-*d₆*) δ 7.94 (s, 1H), 7.84 (s, 1H), 6.17 (s, 1H), 4.47 (s, 2H), 3.83 - 3.79 (m, 2H), 2.67 (s, 2H), 2.41 (s, 3H), 2.35 (s, 3H), 2.22 (s, 3H), 2.17 (s, 3H).

### Biological Assay

### Experimental Example 1

BCA Protein Assay Kit was purchased from Beyotime, Aβ40 and Aβ42 detection kits were purchased from Wako, and cell culture related reagents were purchased from Gibco.

HEK293APP/sw overexpressing cells were cultured in DMEM medium containing 10% FBS, 100 µg/mL G418 (Geneticin) and 1×Penicillin-Streptomycin in 48-well plates. A 4 mM compound stock solution (prepared by dissolving the compound in DMEM culture medium) was filtered through a 0.22 µm sterile filter and stored at -20°C for subsequent use. At 70% cell density, 40 µL of the compound test solution was added to each well to reach a final concentration of 400 µM, and the plate was incubated for 24 hours.

The supernatant of the culture medium was collected. The BCA reagent was added to a part of the supernatant, after incubating at room temperature for 30 min, the absorbance value (i.e., OD value) of each well was determined by a microplate reader at 570 nm. The total protein concentration was calculated according to a protein standard curve. Meanwhile, the other part of the supernatant (100 µL) was added to a coated 96-well plate and incubated overnight at 4°C, after the solution was removed and the reagents were washed, HRP (horseradish peroxidase)-labeled antibody was added and incubated at 4°C for 2 hours. After the reagents were removed and washed, a TMB chromogenic solution was added, the plate was incubated at room temperature for 30 min, and then a stop solution was added to stop the reaction. The absorbance value (i.e., OD value) of each well was measured by a microplate reader at 450 nm. The concentrations of Aβ40 and Aβ42 were calculated according to the standard curves of Aβ40 and Aβ42, respectively. In the end, the concentrations of Aβ40 and Aβ42 were adjusted by the total protein concentration, so as to obtain the final concentration. The test results are shown in the following table.

| **Compound No.** | **The content of Aβ40 (pmol/L)** | **The content of Aβ42 (pmol/L)** |
|---|---|---|
| 1 | 41.18 | 3.32 |
| 2 | 37.39 | 3.34 |
| 5 | 48.94 | 4.92 |
| 7 | 35.93 | 5.03 |
| 8 | 60.96 | 2.31 |
| 9 | 42.88 | 2.94 |
| 13 | 43.76 | 7.21 |
| 15 | 37.88 | 10.52 |
| Blank control* | 127.81 | 11.08 |

| | | |
|---|---|---|
| * No compound test solution was added to the blank control. | | |

According to the above test results, the compounds of the present invention can significantly reduce the levels of Aβ42 and/or Aβ40.

### Experimental Example 2. acute toxicity test

### 2.1. Test purpose

The toxic reaction and death after administration of the test compound to mice by gavage were observed for preliminary evaluation of the safety of the test compound.

### 2.2. Test method

### 2.2.1. Test materials

CMC-Na (sodium carboxymethylcellulose) was purchased from Shanghai Sinopharm Chemical Reagent Co., Ltd.

The test animals were Kunming strain mice, 18-20 g, provided by Beijing Keao Xieli Feed Co., Ltd.

### 2.2.2. Test procedures

1) Preparation of 0.7% CMC-Na: 0.7 g CMC-Na was added with distilled water to prepare a 0.7 g/100 mL solution.
2) The compound to be tested was added to 0.7% CMC-Na to prepare a 100 mg/mL suspension.
3) Each test group comprised 10 mice, all of which were male. Each mouse was orally administered with the test compound (2000 mg/kg) by gavage, once a day, for a cumulative period of 15 days. After administration, animals were observed for the following responses: animal diet, appearance, behavior, secretions, excreta, symptoms of abnormal animal responses, onset time, severity, duration, reversibility, and death. The body weights were recorded on the day of administration, days 7 and 14.

### 2.3. Test results

| Group | Compound No. | Number of animals | Body weight before administration (mean ± standard deviation, g) | Body weight on Day 7 (mean ± standard deviation, g) | Body weight on Day 14 (mean ± standard deviation, g) |
|---|---|---|---|---|---|
| 1 | Compound 1 | 10 | 31.4±1.1 | 35.5±1.0 | 36.7±1.2 |
| 3 | Compound 9 | 10 | 32.1±1.7 | 35.7±1.4 | 36.3±1.7 |

After administration of the test compounds, the animals showed no abnormalities. No significant toxic reactions were noticed during continuous observation, indicating that the test compound was safe.

### Experimental Example 3. water maze behavior test

### 3.1. Test principle

Rodents in water have a strong incentive to escape from the water environment, and can escape from the water environment in the fastest and most direct way. The process of learning to escape from the water environment reflects the learning ability of the animals. Spatial positioning according to the surrounding environment and purposefully swimming to a safe place in the water (such as a platform) can reflect the spatial learning and memory ability of the animals.

### 3.2. Test method

### 3.2.1. Test materials

### 1) Test animals

The test animals were APP/PS1 2×Tg mice (C57BL/6 wild-type mice as the blank control). Mice of 6-8 months old, weighing 20-40 g, were purchased from The Jackson laboratory.

APP/PS1 mice are APP/PS1 di-transgenic Alzheimer's disease (2×Tg-AD) model mice, and the appearance of deposition of Aβ is earlier than the pathological changes of tau protein for several months, which can more truly simulate the clinical process and pathological changes of Alzheimer's disease.

### 2) Main reagents

CMC-Na (sodium carboxymethylcellulose), purchased from Shanghai Sinopharm Chemical Reagent Co., Ltd.;
Positive control: BTMP (benfotiamine), prepared by Shanghai Raising Pharmaceutical Co., Ltd. 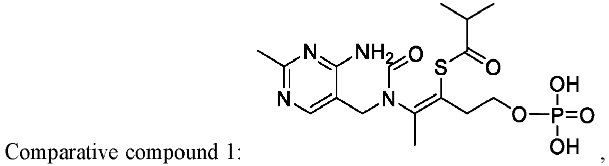 prepared according to the method of Example 6 in CN201811435584.X.

### 3) Main instruments

| Name | Manufacturer | Model/version number | Manufacturing Code |
|---|---|---|---|
| Electronic balance | Mettler Toledo | PL6001E | C041242339 |
| Electronic balance | Fuzhou Huazhi Scientific Instrument Co., Ltd | HZT-A+300 | 18190 |
| DigBehv animal behavior analysis system | Shanghai Jiliang Software Technology Co., Ltd. | 4.023.170106 | / |

### 3.2.2. Test method

### 1) Drug preparation and administration information

### 1.1. Drug preparation

Preparation of 0.7% CMC-Na: 0.7 g CMC-Na was added with a proper amount of water, heated for dissolution, left for cooling to room temperature, diluted to 100 mL, and stored at 4°C.

Preparation of BTMP: 100 mg of BTMP was added to 10 mL of 0.7% CMC-Na to prepare a 10 mg/mL suspension for use.

Preparation of test compound and comparative compound 1: 100 mg of the compound to be tested was weighted and added to 10 mL of 0.7% CMC-Na to prepare a 10 mg/mL suspension for use.

### 1.2. Administration information

The laboratory mice were randomly divided into groups (9 or 10 mice per group). Mice in each group were administrated at a dosage of 0.2 mL/10 g body weight by oral gavage once a day for eight weeks.

| Group | Drug/dosage | Gender | Administration Period |
|---|---|---|---|
| Blank control group | 0.7% CMC-Na | Male/female | 8w |
| Model group | 0.7% CMC-Na | Male/female | 8w |
| BTMP | 200 mg/kg | Male/female | 8w |
| Compound to be tested | 200 mg/kg | Male/female | 8w |
| Comparative compound 1 | 200 mg/kg | Male/female | 8w |

### 2) Test procedures

The mice in the blank control group, model group and test group were orally administrated for 8 consecutive weeks according to the above administration dosage/specification. The water maze training and test were started in the last week of administration. The water maze training and test lasted for six days, with a training period of five days and a testing period of one day. In addition, during the training and testing period of water maze (6 days in total), the indoor conditions such as lighting etc. were kept consistent, the room was kept quiet, and the interference from the environment and personnel was excluded.

2.1. Preparation before the test: a proper amount of water was put into the water maze pool with the water temperature being kept at 22±3°C. The platform was placed in a fixed position (target quadrant) and 1 cm below the water surface, and titanium dioxide was added until the water became white, so that the platform could not be seen clearly.

2.2. Training period (days 1 to 5): on each day, before the first quadrant training, each mouse was placed on the platform for 15s (to increase the mice's sense of security on the platform), and then the mouse was put into the pool (with its head facing the pool wall) from the quadrant where the platform located, and the swimming time was set as 60 s. If the mouse found the platform within 60 s and stayed for 5 s, it was regarded as the platform being found successfully. If the mouse could not find the platform, then the time was recorded as 60s. The mouse was guided to the platform and stayed for 20s, the mouse was then taken out, and the quadrant training for this mouse was ended.

After the first quadrant training, the mouse was trained in the remaining three quadrants sequentially, where it is not necessary to place the mouse on the platform for 15s before training. The training of each mouse in every two quadrants was conducted at an interval of 10-15 minute. In this way, the training was performed continuously for 5 days.

2.3. Testing period (day 6): 24 hours after the last training, the platform was removed, and the mouse was dropped at the position opposite to the quadrant where the platform previously located (i.e., the position furthest away from the platform). The time of the mouse in the target quadrant (the quadrant where the platform previously located), the number of crossing the position where the platform previously located (the number of crossing the platform), and the time of crossing the position where the platform previously located for the first time (the latency period) were recorded, which were used as indicators for evaluating the spatial learning and memory ability of the mouse.

| Group | Number | Latency period (s) | The number of crossing the platform | Time in the target quadrant |
|---|---|---|---|---|
| Blank control group | 10 | 21.8±3.3^{##} | 2.5±0.9^{##} | 20.0±2.3^{##} |
| Model group | 10 | 45.0±8.8^{∗∗} | 0.9±0.6^{∗∗} | 12.8±2.9^{∗∗} |
| BTMP | 10 | 27.3±1.6^{∗##} | 2.1±0.6^{##} | 18.0±3.5^{##} |
| Compound 1 | 10 | 22.0±1.5^{##} | 2.4±0.8^{##} | 19.6±3.4^{##} |
| Compound 2 | 10 | 22.7±1.1^{##} | 2.2±0.6^{##} | 18.8±2.6^{##} |
| Compound 5 | 9 | 25.0±2.2^{##} | 2.2±0.7^{##} | 17.7±2.6^{##} |
| Compound 9 | 9 | 22.8±1.3^{##} | 2.3±0.5^{##} | 18.6±1.6^{##} |
| Comparative compound 1 | 10 | 27.0±1.3^{##} | 2.1±0.6^{##} | 18.1±2.8^{##} |

| | | | | |
|---|---|---|---|---|
| Note: compared with the blank control group, **P<0.01, *P < 0.05; compared with the model group, ##P<0.01, #P<0.05. | | | | |

According to the test results, the latency period of the animals in the test group administered with the compound of the present application (for example, the latency period of the animals in the test group administered with compound 1 was 22.0±1.5 s) was substantially equivalent to that of the animals in the blank control group (21.8±3.3 s), and was much lower than that of animals in the test group administered with BTMP or the Comparative compound 1 (27.3±1.6 s and 27.0±1.3 s, respectively). This effect achieved by the compound of the present application is unexpected.

### Experimental Example 4. Jumping stand test

### 1.1. Test animals and reagents

The mice employed in the present test were purchased from Xipuer-BIKAI Laboratory Animal Co., Ltd., and they were SPF grade ICR mice, 3-4 weeks old, weighing 16-18 g.

Scopolamine hydrobromide trihydrate was purchased from aladdin, Product No.: S107418; Purity: 98%.

Sodium nitrite (NaNO₂) was purchased from Sinopharm Chemical Reagent Co., Ltd.; Product No.: 10020018; Specification: analytical grade.

### 1.2. Drug preparation and administration information

### 1) Drug preparation

Preparation of 0.7% CMC-Na: 0.7 g CMC-Na was weighed and added with a proper amount of purified water, heated for dissolution, left for cooling to room temperature, diluted to 100 mL, and stored at 4°C.

Test article preparation: 100 mg of the compound to be tested was weighed and added to 10 mL of 0.7% CMC-Na to prepare a 10 mg/mL suspension for use.

### 2) Administration information

The laboratory mice were randomly divided into groups (9 or 10 mice per group). The mice in each group were administrated at a dosage of 0.2 mL/10 g body weight by oral gavage once a day for three weeks.

| Group | Drug/dosage | Gender | Administration Period |
|---|---|---|---|
| Blank group | 0.7% CMC-Na | male | 3w |
| Model group | 0.7% CMC-Na | male | 3w |
| Compound to be tested | 200 mg/kg | male | 3w |

### 1.3. Test procedures

The laboratory animals were administered by gavage for 3 consecutive weeks according to the above administration dosage/specification. Training was started on the last day of administration, and the test was conducted 24 hours later.

### 1) Scopolamine-induced acute memory dysfunction model

The laboratory animals were administered by gavage for 3 weeks, and scopolamine (mouse: 2 mg/kg) was injected intraperitoneally 0.5 hour after the last dose. The animals were trained with YLS-3TB jumping stand detector and tested for memory acquisition function after 24 hours. The latency period (the time from the start of counting to the first time of jumping off the stand) and the number of errors (the number of electric shock) were recorded.

### 2) Sodium nitrite-induced hypoxic memory impairment model

The laboratory animals were administered by gavage for three weeks. One hour after the last administration, the animals were trained with YLS-3TB jumping stand detector. After the training, sodium nitrite (mouse: 120 mg/kg) was injected subcutaneously (in the neck) immediately. The jumping stand test was conducted 24 hours later. The latency period (the time from the start of counting to the first time of jumping off the stand) and the number of errors (the number of electric shock) were recorded.

### 1.4. Behavior detection in the jumping stand test

The YLS-3TB jumping stand detector were divided into five compartments by transparent boards. The bottom of the compartment was covered with a copper grid and applied with a voltage of 50 V A rubber pad stand with a height of 3.5 cm and a diameter of 3.5 cm was placed in each compartment to serve as a safe area for mice to avoid electric shock. Training of laboratory mice before the formal test: the laboratory mice were placed in the small compartment with a stand to get familiar with the environment for 5 min (the five compartments were connected), and then electrified for stimulation (all the laboratory mice were on the grid at this time), at the voltage of 50 V and current of 1.00 mA. Mice have the habit of jumping from a high place. If they jump, they will be shocked to produce memory. The current was applied for 5 min, which served as the training process.

Testing: the animals were put on the stand in the jumping compartment one by one. The time interval for putting each animal was 5 s. After the first animal was put on the stand, the electricity was applied immediately. The electricity was applied for 6 min. At the end of applying electricity, data were printed, and the latency period (the time from the start of counting to the first time of jumping off the stand) and the number of errors (the number of electric shock during the testing period) were recorded. Due to the different time of putting the animal in, the time difference can be subtracted according to the actual time of putting the animal.

### Jumping stand test results of scopolamine-induced acute memory dysfunction model

| Group | Number | Latency period (s) | Number of errors |
|---|---|---|---|
| Blank group | 10 | 203.0±116.9^{##} | 0.7±0.82^{##} |
| Model group | 10 | 68.5±80.0^{∗∗} | 1.7±0.5^{∗∗} |
| Compound 1 | 10 | 180.0±105.9^{#} | 0.8±0.8^{##} |
| Compound 2 | 10 | 173.2±98.7^{#} | 0.9±0.7^{#} |

| | | | |
|---|---|---|---|
| Note: compared with the blank control group, **P<0.01, * P < 0.05; compared with the AD model group ##P<0.01, #P<0.05. | | | |

### Jumping stand test results of sodium nitrite-induced hypoxic memory impairment model

| Group | Number | Latency period (s) | Number of errors |
|---|---|---|---|
| Blank group | 10 | 267.4±79.8^{##} | 0.2±0.4^{##} |
| Model group | 10 | 93.8±90.25^{∗∗} | 1.4±0.7^{∗∗} |
| Compound 1 | 10 | 228.8±155.9^{##} | 0.4±0.7^{##} |
| Compound 2 | 10 | 210.4±117.3^{#} | 0.6±0.8^{#} |
| Compound 9 | 9 | 209.3±110.4^{#} | 0.6±0.7^{#} |

| | | | |
|---|---|---|---|
| Note: compared with the blank control group, **P<0.01, * P < 0.05; compared with the AD model group ##P<0.01, #P<0.05. | | | |

### Experimental Example 5. behavioral detection in a dark avoidance test

### 1. Test materials

### 1.1. Test animals and reagents

The mice employed in the present test were purchased from Xipuer-BIKAI Laboratory Animal Co., Ltd., and they were SPF grade ICR mice, 3-4 weeks old, weighing 16-18 g.

Anhydrous ethanol: Shanghai Titan Technology Co., Ltd.; Product No.: G73537B; Purity: ≥ 99.7%.

### 1.2. Drug preparation and information

### 1) Drug preparation

Preparation of 0.7% CMC-Na: 0.7 g CMC-Na was weighed and added with a proper amount of purified water, heated for dissolution, left for cooling to room temperature, diluted to 100 mL, and stored at 4°C.

Test article preparation: 100 mg of the compound to be tested was weighed and added to 10 mL of 0.7% CMC-Na to prepare a 10 mg/mL suspension for use.

### 2) Administration information

The laboratory mice were randomly divided into groups (9 or 10 mice per group). The mice in each group were administrated at a dosage of 0.2 mL/10 g body weight by oral gavage once a day for three weeks.

| Group | Drug/dosage | Gender | Administration Period |
|---|---|---|---|
| Blank group | 0.7% CMC-Na | male | 3w |
| Model group | 0.7% CMC-Na | male | 3w |
| Compound to be tested | 200 mg/kg | male | 3w |

### 2. Memory reproduction disorder model induce by ethanol

The laboratory mice animals were administered by gavage for three weeks. One hour after the last administration, they were trained with the YLS-17B dark avoidance tester. The test was performed 24 h later. A 45% ethanol solution (0.1 mL/10 g) was administered by oral gavage 30 min before the test. The latency period (the time from the start of counting to the first time of entering the dark compartment) and the number of errors (number of electric shocks) of the animals were recorded.

The activity box of the dark avoidance automatic tester comprises two compartments (bright and dark), there is a hole between the two compartments, the bottom of the box is covered by a copper grid, and the animals normally go into the dark compartment. Before the formal test, the laboratory mice were trained by placing the animals in the bright compartment with their backs facing the entrance of the hole, to get familiar with the environment for 5 min, and then the copper grid in the dark compartment was electrified for stimulation at a voltage of 50 V and a current of 1.00 mA (which can be adjusted according to the actual situation). When the animals received electric shock, they entered the bright compartment, or went back and forth between the two compartments. The current was continuously applied for 5 min, which served as the training process.

Testing: the mice modeled with ethanol were put into the bright box one by one with their backs facing the entrance of the hole, and the time interval between putting each animal was 5 s. After the first animal was put into the bright compartment, the electricity was applied immediately. The electricity was applied for 6 min. At the end of applying electricity, data were printed, and the latency period (the time from the start of counting to the first time of entering the dark compartment) and the number of errors (the number of electric shock during the testing period) were recorded. Due to the different time of putting the animal in, the time difference can be subtracted according to the actual time of putting the animal. If the mice did not enter the dark compartment within 5 min, the latency period was recorded as 300s s.

Statistical analysis: all data were expressed as the mean value ± standard deviation, and SPSS software was used for analysis.

### Dark avoidance test results of memory reproduction disorder model induce by ethanol

| Group | Number | Latency period (s) | Number of errors |
|---|---|---|---|
| Blank group | 10 | 232.2±117.1^{#} | 0.3±0.48^{##} |
| Model group | 10 | 118.4±106.9^{∗} | 2.4±1.7^{∗∗} |
| Compound 1 | 10 | 229.8±90.0^{#} | 1.0±1.25 |
| Compound 2 | 10 | 217.4±96.6^{#} | 1.2±1.3 |
| Compound 9 | 9 | 216.1±76.8^{#} | 1.0±1.0^{#} |

| | | | |
|---|---|---|---|
| Note: compared with the Blank group, **P<0.01, *P < 0.05; compared with the AD model group ##P<0.01, #P<0.05. | | | |

### Experimental Example 6 pharmacokinetic study

### 1.1 Laboratory animals and reagents

The SD rats used in this test were purchased from Zhejiang Charles rive Laboratory Animal Co., Ltd. Both male and female rats of SPF grade weighting about 250 g were used.

Sodium carboxymethyl cellulose (CMC-Na) was obtained from Sinopharm Chemical Reagent Co., Ltd. Product No.: 20160704.

BTMP is prepared by Shanghai Raising Pharmaceutical Co., Ltd. 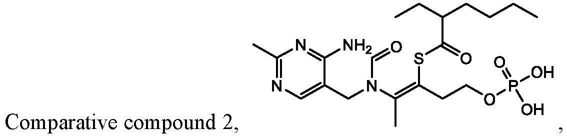 was prepared according to the method described in Example 13 in CN201811435584.X.

### 1.2 Drug preparation

Preparation of 0.7% CMC-Na: 7 g CMC-Na was accurately weighed and put into a 1 L beaker. A magnetic stirrer was put into the beaker, and then 1 L purified water was added. The mixture was stirred until a transparent solution was obtained.

Preparation of the test article: a proper amount of test article was weighed and ground as fine powder in a mortar. A certain amount of the vehicle was added to a proper container, and then the ground test article was added to the container under magnetic stirring. The mixture was stirred until the appearance is uniform. The remaining vehicle was gradually added, followed by magnetic stirring for at least 20 min, so as to prepare a solution at a concentration of 15.353 mM.

### 1.3 Test procedures

### (1) Absorption test

The SD rats were divided into 3 groups (6 rats in each group, half male and half female). Animals in each group were single gavage administrated with Compound 1 (75 mg/kg free base, 15.353 mM), BTMP (71.6 mg/kg, 15.353 mM), or Comparative compound 2 (75 mg/kg free base, 15.353 mM), respectively. Blood was collected (about 200 µL/time) before drug administration (0 min) and 3 min, 8 min, 15 min, 30 min, 1 h, 2 h, 3 h, 5 h, 8 h, 12 h, 24 h and 48 h after drug administration. Blood was anticoagulated with EDTA and placed on ice after collection. For the blood samples collected at each time point, 150 µL of whole blood was taken and 150 µL 5.2% perchloric acid was added to determine TM (Thiamine) and TDP (Thiamine diphosphate) (TM and TDP were the in vivo metabolites of Compound 1, BTMP, and Comparative compound 2). All samples were frozen and stored in a -80°C refrigerator.

### (2) Brain distribution test

The male SD rats were divided into three groups (16 rats in each group) (4 animals were sacrificed at 4 time points). Animals in each group were single gavage administrated with either Compound 1 (75 mg/kg free base, 15.353 mM), BTMP (71.6 mg/kg, 15.353 mM), or Comparative compound 2 (75 mg/kg free base, 15.353 mM), respectively. The animals were sacrificed at 5 min, 1 h, 3 h, and 9 h after the administration. The brain was taken out immediately after sacrifice, rinsed with ice-cold water and rinsed. 50 mg of the brain tissue was weighed, added to 0.45 mL of 100 mM dipotassium hydrogen phosphate buffer (pH=5.0) and homogenized. Blood samples were taken simultaneously and anticoagulated with EDTA. After collection, blood samples were placed on ice. 150 µL of whole blood was taken and 150 µL of 5.2% perchloric acid was added to determine TM. Another 4 animals were gavage administered with normal saline for the determination of endogenous TM concentration. All samples were frozen and stored in a -80°C refrigerator.

### (3) Sample assay

The concentrations of TM and TDP in the samples were determined by HPLC-FLD.

### (4) Data processing

The non-compartment model of the DAS software was used to calculate the pharmacokinetic parameters of TM and TDP in blood of rats after administration. The concentration of TM in the brain tissue after the drug administration in rats was measured, and the mean value and standard deviation were calculated. The area under the drug-time curve AUC was calculated using the trapezoidal method.

### 1.4 Test results

### 1.4.1 Plasma concentration and pharmacokinetic parameters of rats in the absorption test

A summary of the pharmacokinetic parameters of compound 1, BTMP, and Comparative compound 2 after a single gavage administration to the rats is presented in Table 1.

**Table 1 Pharmacokinetic parameters of rats after oral administration (calculated after deducting concentration at time zero, Mean±SD, n=6)**

| Mode of administration | Component | AUC₀₋ₜ | AUC_{0-∞} | t_{1/2} | Tmax | Cₘₐₓ |
|---|---|---|---|---|---|---|
| | | ng·h/mL | ng·h/mL | h | h | ng/mL |
| Compound 1 | TM | 166847.3 ± | 167175.3 ± | 2.35 ± | 1.33 ± | 28219.5 ± |
| | | 92045.4 | 92540.5 | 0.39 | 0.52 | 14339.7 |
| | TDP | 40066.8 ± 13867.2 | 42484.6 ± 14360.1 | 5.38 ± 1.13 | 4.33 ± 1.03 | 2987.9 ± 812.6 |
| BTMP | TM | 92966.9 ± 22216.1 | 93093.5 ± 22150.2 | 2.41 ± 0.32 | 0.75 ± 0.27 | 18442.7 ± 5439.6 |
| | TDP | 31071.2 ± 6309.5 | 32922.2 ± 3076.1 | 3.47 ± 1.68 | 5.00 | 2630.3 ± 347.7 |
| Comparative compound 2 | TM | 51877.9 ± 11723.9 | 51962.4 ± 11745.4 | 2.39 ± 0.43 | 1.08 ± 0.49 | 10776.4 ± 1742.9 |
| | TDP | 29471.8 ± 5184.3 | 29632.2 ± 5223.3 | 2.76 ± 0.56 | 4.67 ± 0.82 | 2742.4 ± 135.5 |

According to the above test results, at the same drug dose, the TM and TDP exposures of the compound 1 administration group were higher than those of BTMP and Comparative compound 2 administration group. Moreover, in terms of t_{1/2} of TDP, t_{1/2} of the compound 1 administration group was significantly higher than t_{1/2} of the BTMP or Comparative compound 2 administration group.

### 1.4.2 Concentration variations in rat brain and whole blood at different times in the brain distribution test

The TM concentrations in brain and whole blood at different times after a single gavage administration of Compound 1, BTMP, and Comparative compound 2 to rats are shown in Table 2.

**Table 2 TM concentration in brain and whole blood after gavage administration to rats (mean±SD, n=4)**

| Time after administration (h) | Concentration in the brain tissue (ng/g) | | | Concentration in the whole blood (ng/mL) | | |
|---|---|---|---|---|---|---|
| | Compound 1 | BTMP | Comparative compound 2 | Compound 1 | BTMP | Comparative compound 2 |
| 0 | 154.0 ± 9.9 | 154.0 ± 9.9 | 154.0 ± 9.9 | 62.8 ± 10.1 | 62.8 ± 10.1 | 62.8 ± 10.1 |
| 0.083 | 153.5 ± 10.8 | 173.7 ± 18.6 | 156.7 ± 19.2 | 661.3 ± 172.9 | 585.4 ± 290.3 | 376.1 ± 162.1 |
| 1 | 336.6 ± 37.9 | 280.7 ± 25.2 | 273.2 ± 24.6 | 19201.7 ± 4352.8 | 12360.2 ± 2999.4 | 8518.7 ± 3180.3 |
| 3 | 333.3 ± 82.0 | 260.0 ± 20.0 | 240.8 ± 12.3 | 17231.5 ± 8565.8 | 10001.2 ± 1669.1 | 5110.8 ± 1446.7 |
| 9 | 211.6 ± 13.7 | 194.3 ± 26.4 | 165.1 ± 9.0 | 3592.5 ± 607.9 | 832.4 ± 418.9 | 258.3 ± 86.2 |

According to the above data, the AUC of TM in the brain after gavage administration of Compound 1, BTMP, and Comparative compound 2 to rats (calculated after deducting the concentration at time zero) was 1156.6 ng h/g, 739.9 ng h/g, and 555.9 ng h/g, respectively. The AUC of TM of the compound 1 administration group was 1.56 times of that of BTMP administration group and 2.08 times of that of the Comparative compound 2 administration group.

Various modifications of the invention in addition to those described herein will become apparent to those skilled in the art from the foregoing description. Such modifications are intended to fall within the scope of the appended claims. Each reference, including all patents, applications, journal articles, books and any other disclosure, referred to herein is hereby incorporated by reference in its entirety.

## Claims

1. A compound or a pharmaceutically acceptable salt, ester, stereoisomer, tautomer, polymorph, solvate, metabolite, isotopically labeled compound or prodrug thereof, wherein the compound has the structure of Formula (I): 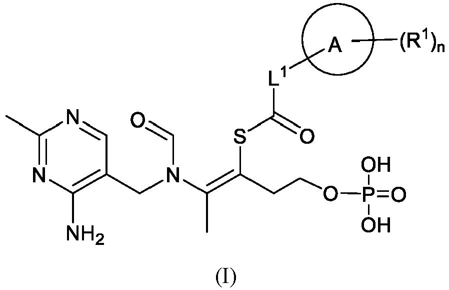 wherein:
ring A is a C₆₋₁₀ aromatic ring or 5- to 14-membered heteroaromatic ring;
L¹ is a direct bond or -R²-C₁₋₆ alkylene-;
R² is -O-, -NH-, -S-, -S(=O)- or -S(=O)₂-;
R¹, at each occurrence, is each independently selected from the group consisting of halogen, hydroxyl, oxo, amino, cyano, nitro, C₁₋₆ alkyl, C₂₋₆ alkenyl, C₂₋₆ alkynyl, C₃₋₆ cyclic hydrocarbyl, 3- to 10-membered heterocyclyl, C₆₋₁₀ aryl, 5- to 14-membered heteroaryl, C₆₋₁₂ aralkyl, =N-OR³, -C(=NH)NH₂, -C(=O)R³, -OC(=O)R³, -C(=O)OR³, -OR³, -SR³, -S(=O)R³, -S(=O)₂R³, -S(=O)₂NR³R⁴, -NR³R⁴, -C(=O)NR³R⁴, -NR³-C(=O)R⁴, -NR³-C(=O)OR⁴, -NR³-S(=O)₂-R⁴, -NR³-C(=O)-NR³R⁴, -C₁₋₆ alkylene-NR³R⁴, -O-C₁₋₆ alkylene-NR³R⁴ and -C₁₋₆ alkylene-O-C₁₋₆ alkyl; or when n is greater than 1, two R¹ together with the group to which they are attached form a C₃₋₆ hydrocarbon ring, 3- to 10-membered heterocycle, C₆₋₁₀ aromatic ring or 5- to 14-membered heteroaromatic ring;
R³ and R⁴, at each occurrence, are each independently selected from the group consisting of H, C₁₋₆ alkyl, C₃₋₁₀ cyclic hydrocarbyl, 3- to 10-membered heterocyclyl, C₆₋₁₀ aryl, 5- to 14-membered heteroaryl and C₆₋₁₂ aralkyl;
the above alkyl, alkylene, alkenyl, alkynyl, cyclic hydrocarbyl, hydrocarbon ring, heterocyclyl, heterocycle, aryl, aromatic ring, heteroaryl, heteroaromatic ring and aralkyl, at each occurrence, are each optionally substituted with one or more substituents independently selected from the group consisting of: halogen, hydroxyl, oxo, amino, cyano, nitro, C₁₋₆ alkyl, C₂₋₆ alkenyl, C₂₋₆ alkynyl, C₃₋₆ cyclic hydrocarbyl, 3- to 10-membered heterocyclyl, C₆₋₁₀ aryl, 5- to 14-membered heteroaryl, C₆₋₁₂ aralkyl, =N-OR⁵, -C(=NH)NH₂, -C(=O)R⁵, -OC(=O)R⁵, -C(=O)OR⁵, -OR⁵, -SR⁵, -S(=O)R⁵, -S(=O)₂R⁵, -S(=O)₂NR⁵R⁶, -NR⁵R⁶, -C(=O)NR⁵R⁶, -NR⁵-C(=O)R⁶, -NR⁵-C(=O)OR⁶, -NR⁵-S(=O)₂-R⁶, -NR⁵-C(=O)-NR⁵R⁶, -C₁₋₆ alkylene-NR⁵R⁶, -O-C₁₋₆ alkylene-NR⁵R⁶ and -C₁₋₆ alkylene-O-C₁₋₆ alkyl, the alkyl, alkenyl, alkynyl, cyclic hydrocarbyl, heterocyclyl, aryl, heteroaryl and aralkyl are further optionally substituted with one or more substituents independently selected from the group consisting of: halogen, hydroxyl, oxo, amino, cyano, nitro, C₁₋₆ alkyl, C₃₋₆ cyclic hydrocarbyl, 3- to 10-membered heterocyclyl, C₆₋₁₀ aryl, 5- to 14-membered heteroaryl and C₆₋₁₂ aralkyl;
R⁵ and R⁶, at each occurrence, are each independently selected from the group consisting of H, C₁₋₆ alkyl, C₃₋₁₀ cyclic hydrocarbyl, 3- to 10-membered heterocyclyl, C₆₋₁₀ aryl, 5- to 14-membered heteroaryl and C₆₋₁₂ aralkyl; and
n is an integer of 0, 1, 2, 3 or 4;
provided that, when L' is a direct bond, ring A is not a benzene ring.

2. The compound according to claim 1, or a pharmaceutically acceptable salt, ester, stereoisomer, tautomer, polymorph, solvate, metabolite, isotopically labeled compound or prodrug thereof, wherein ring A is a benzene ring or 5- to 6-membered heteroaromatic ring.

3. The compound according to claim 2, or a pharmaceutically acceptable salt, ester, stereoisomer, tautomer, polymorph, solvate, metabolite, isotopically labeled compound or prodrug thereof, wherein ring A is a benzene ring, pyrrole ring, furan ring, thiophene ring or pyridine ring.

4. The compound according to any one of claims 1-3, or a pharmaceutically acceptable salt, ester, stereoisomer, tautomer, polymorph, solvate, metabolite, isotopically labeled compound or prodrug thereof, wherein L' is a direct bond or -O-C₁₋₆ alkylene-.

5. The compound according to claim 4, or a pharmaceutically acceptable salt, ester, stereoisomer, tautomer, polymorph, solvate, metabolite, isotopically labeled compound or prodrug thereof, wherein L' is a direct bond, -O-CH₂- or -O-CH₂CH₂-.

6. The compound according to any one of claims 1-5, or a pharmaceutically acceptable salt, ester, stereoisomer, tautomer, polymorph, solvate, metabolite, isotopically labeled compound or prodrug thereof, wherein R¹ is halogen, C₁₋₆ alkyl, C₆₋₁₀ aryl optionally substituted with halogen or -C₁₋₆ alkylene-O-C₁₋₆ alkyl; or two R¹ together with the group to which they are attached form a C₆₋₁₀ aromatic ring, which is optionally further substituted with -OR⁵.

7. The compound according to claim 6, or a pharmaceutically acceptable salt, ester, stereoisomer, tautomer, polymorph, solvate, metabolite, isotopically labeled compound or prodrug thereof, wherein R¹ is -Cl, methyl, phenyl optionally substituted with F or -CH₂-O-CH₃; or two R¹ together with the group to which they are attached form a benzene ring, which is optionally further substituted with methoxy.

8. The compound according to any one of claims 1-7, or a pharmaceutically acceptable salt, ester, stereoisomer, tautomer, polymorph, solvate, metabolite, isotopically labeled compound or prodrug thereof, wherein

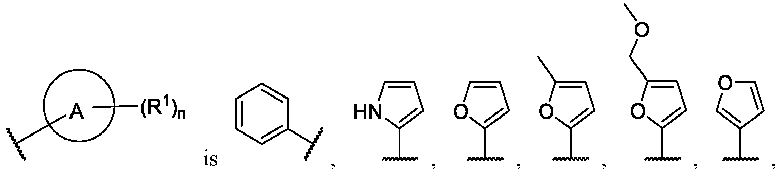

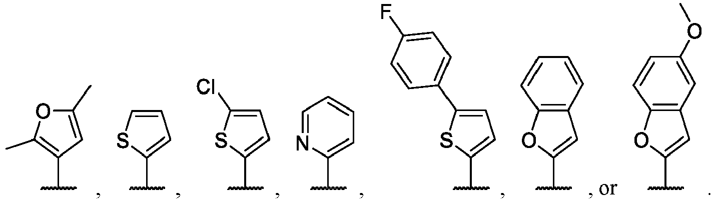

9. A compound or a pharmaceutically acceptable salt, ester, stereoisomer, tautomer, polymorph, solvate, metabolite, isotopically labeled compound or prodrug thereof, wherein the compound is:
| **No.** | **Compound Name** |
|---|---|
| 1 | (Z)-S-(2-(N-((4-amino-2-methylpyrimidin-5-yl)methyl)formamido)-5-(phosphonooxy)pent-2-en-3-yl) 2-propylpentanethioate |
| 2 | (Z)-S-(2-(N-((4-amino-2-methylpyrimidin-5-yl)methyl)formamido)-5-(phosphonooxy)pent-2-en-3-yl) 2-ethylbutanethioate |
| 5 | (Z)-S-(2-(N-((4-amino-2-methylpyrimidin-5-yl)methyl)formamido)-5-(phosphonooxy)pent-2-en-3-yl) O-phenethyl carbonothioate |
| 6 | (Z)-S-(2-(N-((4-amino-2-methylpyrimidin-5-yl)methyl)formamido)-5-(phosphonooxy)pent-2-en-3-yl) O-benzylcarbonothioate |
| 7 | (Z)-S-(2-(N-((4-amino-2-methylpyrimidin-5-yl)methyl)formamido)-5-(phosphonooxy)pent-2-en-3-yl) 5-chlorothiophene-2-carbothioate |
| 8 | (Z)-S-(2-(N-((4-amino-2-methylpyrimidin-5-yl)methyl)formamido)-5-(phosphonooxy)pent-2-en-3-yl) 5-methoxybenzofuran-2-carbothioate |
| 9 | (Z)-S-(2-(N-((4-amino-2-methylpyrimidin-5-yl)methyl)formamido)-5-(phosphonooxy)pent-2-en-3-yl) furan-2-carbothioate |
| 10 | (Z)-S-(2-(N-((4-amino-2-methylpyrimidin-5-yl)methyl)formamido)-5-(phosphonooxy)pent-2-en-3-yl) 1H-pyrrole-2-carbothioate |
| 11 | (Z)-S-(2-(N-((4-amino-2-methylpyrimidin-5-yl)methyl)formamido)-5-(phosphonooxy)pent-2-en-3-yl) 5-(4-fluorophenyl)thiophene-2-carbothioate |
| 12 | (Z)-S-(2-(N-((4-amino-2-methylpyrimidin-5-yl)methyl)formamido)-5-(phosphonooxy)pent-2-en-3-yl) 5-(methoxymethyl)furan-2-carbothioate |
| 13 | (Z)-S-(2-(N-((4-amino-2-methylpyrimidin-5-yl)methyl)formamido)-5-(phosphonooxy)pent-2-en-3-yl) benzofuran-2-carbothioate |
| 14 | (Z)-S-(2-(N-((4-amino-2-methylpyrimidin-5-yl)methyl)formamido)-5-(phosphonooxy)pent-2-en-3-yl) thiophene-2-carbothioate |
| 15 | (Z)-S-(2-(N-((4-amino-2-methylpyrimidin-5-yl)methyl)formamido)-5-(phosphonooxy)pent-2-en-3-yl) furan-3-carbothioate |
| 16 | (Z)-S-(2-(N-((4-amino-2-methylpyrimidin-5-yl)methyl)formamido)-5-(phosphonooxy)pent-2-en-3-yl) pyridine-2-carbothioate |
| 17 | (Z)-S-(2-(N-((4-amino-2-methylpyrimidin-5-yl)methyl)formamido)-5-(phosphonooxy)pent-2-en-3-yl) 5-methylfuran-2-carbothioate |
| 18 | (Z)-S-(2-(N-((4-amino-2-methylpyrimidin-5-yl)methyl)formamido)-5-(phosphonooxy)pent-2-en-3-yl) 2,5-dimethylfuran-3-carbothioate. |

10. A pharmaceutical composition comprising a prophylactically or therapeutically effective amount of the compound according to any one of claims 1-9 or a pharmaceutically acceptable salt, ester, stereoisomer, tautomer, polymorph, solvate, metabolite, isotopically labeled compound or prodrug thereof and one or more pharmaceutically acceptable carriers.

11. Use of the compound according to any one of claims 1-9 or a pharmaceutically acceptable salt, ester, stereoisomer, tautomer, polymorph, solvate, metabolite, isotopically labeled compound or prodrug thereof or the pharmaceutical composition according to claim 10 in the manufacture of a medicament for the prophylaxis or treatment of a neurodegenerative disease or the alleviation of a symptom of the neurodegenerative disease.

12. The use according to claim 11, wherein the neurodegenerative disease is selected from the group consisting of Alzheimer's disease, Creutzfeldt-Jakob disease, Huntington's disease, multiple sclerosis, Guillain-Barre syndrome, Parkinson's disease, Lou Gehrig's disease, paralytic dementia caused by gradual nerve cell death and diseases caused by progressive incontinentia; preferably Alzheimer's disease.

13. A method for preparing the compound of Formula (I): 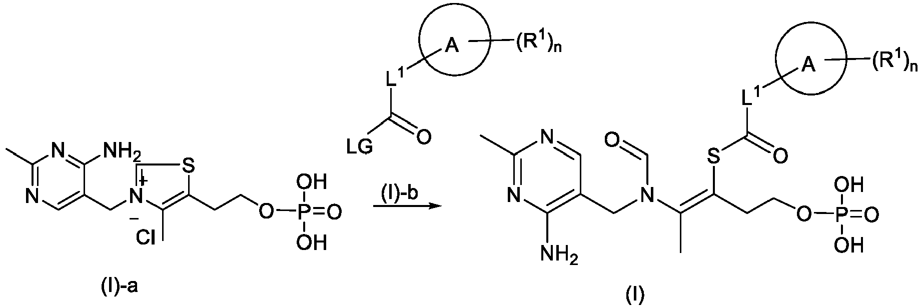 wherein:
LG is a leaving group, preferably halogen, most preferably chlorine;
the remaining groups are as defined in any one of claims 1-9;
the method comprises reacting a compound of formula (I)-a with a compound of formula (I)-b to obtain the compound of formula (I).
